# EUROPÄISCHE PATENTSCHRIFT

(11) **EP 1 185 531 B1**
(45) Veröffentlichungstag und Bekanntmachung des Hinweises auf die Patenterteilung: **29.10.2003**
(21) Anmeldenummer: 01949087.9
(22) Anmeldetag: 25.01.2001
(51) Int. Cl.: C07D 487/04, C09B 57/00, A61K 7/13

(54) **KATIONISCHE PYRROLO-PYRROL-DERIVATE, VERFAHREN ZU DEREN HERSTELLUNG UND DIESE VERBINDUNGEN ENTHALTENDE FÄRBEMITTEL FÜR KERATINFASERN**
CATIONIC PYRROLO-PYRROL-DERIVATIVES, METHOD FOR PRODUCING THEM AND COLORANTS FOR KERATIN FIBRES, CONTAINING THESE COMPOUNDS
DERIVES PYRROLO-PYRROL CATIONIQUES, LEUR PROCEDE DE PREPARATION, ET MATIERE COLORANTE CONTENANT CES COMPOSES, DESTINEE A DES FIBRES DE KERATINE

(30) Priorität: 25.02.2000 DE 10009070
(43) Veröffentlichungstag der Anmeldung: 13.03.2002
(73) Patentinhaber: Wella Aktiengesellschaft, 64274 Darmstadt (DE)
(72) Erfinder: PASQUIER, Cécile, CH-1723 Marly (CH); WYSS, Patrick, CH-1721 Cormérod (CH); BRAUN, Hans-Jürgen, CH-3182 Überstorf (CH)
(86) Internationale Anmeldenummer: EP0100784
(87) Internationale Veröffentlichungsnummer: WO01062759

(56) Entgegenhaltungen:
- EP-A- 0 953 343
- DE-A- 4 011 927
- DE-A- 4 037 556
- US-A- 5 169 953

## Beschreibung

Gegenstand der vorliegenden Erfindung sind neue kationische Pyrrolo-pyrrol-Derivate, ein Verfahren zu deren Herstellung sowie diese Derivate enthaltende Färbemittel für Keratinfasern, insbesondere menschliche Haare.

Für die farbverändemde Behandlung keratinhaltiger Fasern, zum Beispiel menschlicher Haare, Wolle oder Pelzen, werden in der Regel zwei Färbeverfahren angewendet. Im ersten Verfahren wird die Färbung mit sogenannten oxidativen oder permanenten Färbemitteln unter Verwendung einer Mischung aus verschiedenen Entwicklersubstanzen und Kupplersubstanzen und eines Oxidationsmittels erzeugt. Bei Bedarf können zur Abrundung des Färbeergebnisses oder zur Erzeugung von besonderen Farbeffekten sogenannte direktziehende (nicht-oxidative) Farbstoffe zugesetzt werden. Das zweite Verfahren bedient sich ausschliesslich direktziehender Farbstoffe, die in einer geeigneten Trägermasse auf die Fasern aufgebracht werden. Dieses Verfahren ist einfach anzuwenden, ausgesprochen mild und zeichnet sich durch eine geringe Schädigung der Keratinfaser aus. An die hierbei verwendeten direktziehenden Farbstoffe werden eine Vielzahl von Anforderungen gestellt. So müssen sie in toxikologischer und dermatologischer Hinsicht unbedenklich sein und die Erzielung von Färbungen in der gewünschten Intensität ermöglichen, was unter anderem auch eine ausreichende Wasserlöslichkeit voraussetzt. Außerdem wird für die erzielten Färbungen eine gute Lichtechtheit, Säureechtheit und Reibechtheit gefordert.

Für ein direktziehendes (nicht-oxidatives) Färbemittel für Keratinfasern wird in der Regel eine Kombination von verschiedenen nicht-oxidativen Farbstoffen benötigt. Da die Auswahl an in Färbemitteln für Keratinfasem einsetzbaren roten und blauen Farbstoffen beschränkt ist, besteht weiterhin ein Bedarf an derartigen Farbstoffen.

Wasserlösliche Diketopyrrolo[3,4-c]pyrrole, welche mindestens eine wasserlöslich-machende anionische Gruppe enthalten, sind aus der DE-OS 40 11 927 bekannt. Diese Verbindungen sollen einheitliche, brilliante Färbungen von stickstoffhaltigen und cellulosischen Fasern mit guten Echtheitseigenschaften ermöglichen, wobei derartige Verbindungen wegen ihres schlechteren Aufziehverhaltens auf Keratinfasem in der Regel nur in Verbindung mit Carriern befriedigende Färbungen ergeben. Weiterhin ist es aus der EP-OS 0 953 343 bekannt, wasserunlösliche Diketopyrrolo[3,4-c]pyrrole als färbende Pigmente in kosmetischen Mitteln, wie zum Beispiel Schminkpräparaten, einzusetzen. Ebenfalls sind aus der DE-OS 44 35 211 kationische Diketopyrrole bekannt, welche als elektrochrome Materialien in Anzeige-Systemen Verwendung finden sollen.

Es wurde nunmehr gefunden, dass durch die Einführung einer kationischen Gruppe hervorragend wasserlösliche Diketopyrrolo[3,4-c]pyrrole erhalten werden, welche die vorgenannten Anforderungen weitestgehend erfüllen und ohne den Zusatz von Carriem intensive Färbungen mit guter Waschstabilität, Lichtstabilität und Schweißstabilität ermöglichen.

Gegenstand der vorliegenden Erfindung sind daher kationische Diketopyrrolopyrrole der allgemeinen Formel (I) 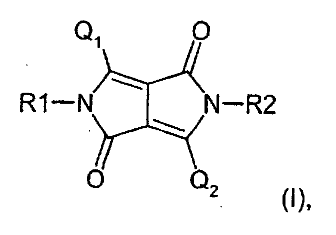 worin unabhängig voneinander Q₁ und Q₂ einen gegebenenfalls substituierten aromatischen isozyklischen oder heterozyklischen Rest mit 5 bis 14 Atomen im Ring, wobei der heterozyklische Rest mindestens ein Sauerstoff-, Stickstoff- oder Schwefelatom enthält, vorzugsweise einen aromatischen monozyklischen bis trizyklischen Rest, insbesondere einen aromatischen monozyklischen oder bizyklischen Rest mit 6 bis 10 Atomen, bedeuten und die Reste R1 und R2 unabhängig voneinander Wasserstoff oder eine Gruppe Y-B⁺ A⁻ bedeuten, unter der Bedingung, dass mindestens einer der Reste R1 und R2 nicht gleich Wasserstoff ist; B⁺ gleich einer aromatischen, aliphatischen, alizyklischen, aromatischen heterozyklischen oder nicht-aromatischen heterozyklischen, quatemären Ammoniumgruppe oder quatemären Phosphoniumgruppe ist; Y gleich einer gegebenenfalls substituierten, geradkettigen oder verzweigten C₁-bis C₆-Alkylengruppe ist und A⁻ gleich einem Anion ist.

In der allgemeinen Formel (I) steht B⁺ vorzugsweise für
(i) eine aromatische, heterozyklische quatemäre Ammoniumverbindung, insbesondere eine quaternäre Verbindung des N-Methylimidazols, N-Allylimidazols, 2-Ethylimidazols oder 1,2-Dimethylimidazols oder eine quaternäre Verbindung des Pyridins, 4-Dimethylaminopyridins, Pyrimidins, Pyrazols, N-Methyl-pyrazols oder Chinolins; oder
(ii) eine nicht-aromatische heterozyklische quaternäre Ammoniumverbindung, insbesondere eine quaternäre Verbindung des Diazabicyclo[2,2,2]octans, N-Methyl-morpholins, N-Ethylmorpholins, 1-Methylpiperidins oder Urotropins; oder
(iii) eine quaternäre Alkylammoniumverbindung, Arylammoniumverbindung oder Arylalkylammoniumverbindung der Formel NR⁵R⁶R⁷, wobei R⁵,R⁶ und R⁷ unabhängig voneinander einen Benzylrest, einen Phenylrest, oder einen C₁- bis C₆- Alkylrest, insbesondere einen Methylrest, einen Ethylrest, einen Propylrest, einen Isopropylrest oder einen Butylrest, bedeuten, wobei die vorgenannten Alkylreste unsubstituiert oder mit einer oder mehreren Hydroxygruppen oder Aminogruppen substituiert sein können; oder
(iv) eine quaternäre Phosphoniumverbindung, insbesondere eine Trialkylphosphoniumverbindung oder Triarylphosphoniumverbindung.
Besonders bevorzugt steht B⁺ hierbei für eine N-Methylimidazoliumverbindung, eine 4-Dimethylaminopyridiniumverbindung, eine Triethylammoniumverbindung oder eine Trimethylammoniumverbindung.

In der allgemeinen Formel (I) stellt Y vorzugsweise eine -CH₂-Gruppe, eine -CH₂-CH₂-Gruppe, eine -CH₂-CH₂-CH₂-Gruppe, eine -CH₂-CH₂-CH₂-CH₂-Gruppe oder einen mit einer oder mehreren Alkylgruppen, Hydroxygruppen, Aminogruppen, Acylgruppen oder quatemären Ammoniumgruppen substituierten, geradkettigen oder verzweigten C₂- bis C₆- Alkylen rest, insbesondere einen substituierten -CH₂-CH₂-Rest, -CH₂-CH₂-CH₂-Rest oder -CH₂-CH₂-CH₂-CH₂-Rest, beispielsweise einen -CH₂-CH(OH)-CH₂-Rest, dar, wobei die -CH₂-CH₂-CH₂-CH₂-Gruppe besonders bevorzugt ist.

Besonders bevorzugt sind Verbindungen der Formel (I), in denen Q₁ und Q₂ unabhängig voneinander die folgende Bedeutung haben:
Phenyl, Biphenyl, 3- Pyridyl , 4-Pyridyl, C₆H₄R3 oder C₆H₃R3R4, mit R3 und R4 unabhängig voneinander gleich F, Cl, Br, J, CN, NO₂, CF₃, C₁-bis C₄-Alkyl, C₁-bis C₄-Alkoxy, Aryloxy, C₁-bis C₄-Thioalkyl oder NR'R", wobei R' und R" unabhängig voneinander gleich Wasserstoff oder einer C₁-bis C₄- Alkylgruppe sind, oder R' und R" gemeinsam mit dem N-Atom einen aromatischen oder aliphatischen Ring mit 4 bis 6 Atomen bilden, der gegebenenfalls ein weiteres Stickstoff-, Sauerstoff- oder Schwefel-Atom enthält.

Unter den vorgenannten Verbindungen der Formel (I) sind solche ganz besonders bevorzugt, bei denen Q₁ und Q₂ unabhängig voneinander gleich Phenyl, Biphenyl, C₆H₄R3 oder C₆H₃R3R4 sind, mit R3 und R4 unabhängig voneinander gleich F, Cl, Br, CN, C₁-bis C₄-Alkyl, C₁-bis C₄-Alkoxy, C₁-bis C₄-Thioalkyl oder NR'R", wobei R' und R" unabhängig voneinander gleich Wasserstoff oder einer C₁-bis C₄-Alkylgruppe sind, oder R' und R" gemeinsam mit dem N-Atom einen aromatischen oder aliphatischen Ring mit 4 bis 6 Atomen bilden, der gegebenenfalls ein weiteres Stickstoff-, Sauerstoff- oder Schwefel-Atom enthält.

In der allgemeinen Formel (I) steht A- vorzugsweise für ein Chlorid-Anion, Bromid-Anion oder lodid-Anion. Es ist jedoch auch möglich, anstelle eines Halogenid-Anions andere Anionen, welche sowohl einwertig als auch mehrwertig sein können, wie zum Beispiel ein Sulfat-Anion, Phosphat-Anion, Hydrogenphosphat-Anion, Carbonat-Anion, Hydrogencarbonat-Anion, Oxalat-Anion, Formiat-Anion, Acetat-Anion, Zitrat-Anion, Tartrat-Anion, Malonat-Anion oder Pyruvat-Anion, einzusetzen, wobei die Zahl der Anionen im Falle von mehrwertigen Anionen durch die Wertigkeit des verwendeten Anions geteilt werden muß. Besonders bevorzugt sind das Bromid-Anion und das Chlorid-Anion.

Als geeignete kationische Diketopyrrolopyrrole der allgemeinen Formel (I) können beispielsweise genannt werden: 2,5-Bis(4-Trimethylammoniumbutyl)-3,6-diphenyl-pyrrolo[3,4-c]pyrrol-1,4-dion-dibromid; 2,5-Bis(3-Trimethylammonium-propyl)-3,6-diphenyl-pyrrolo[3,4-c]pyrrol-1,4-diondibromid; 2,5-Bis(2-Trimethylammonium-ethyl)-3,6-diphenyl-pyrrolo-[3,4-c]pyrrol-1,4-dion-dibromid; 2,5-Bis(4-Trimethylammonium-butyl)-3,6-diphenyl-pyrrolo[3,4-c]pyrrol-1,4-dion-dichlorid; 2,5-Bis(3-Trimethylammonium-propyl)-3,6-diphenyl-pyrrolo[3,4-c]pyrrol-1,4-dion-dichlorid; 2,5-Bis(2-Trimethylammonium-ethyl)-3,6-diphenyl-pyrrolo-[3,4-c]pyrrol-1,4-dion-dichlorid; 2,5-Bis[4-[4-(Dimethylamino)pyridinium]butyl]-3,6-diphenyl-pyrrolo[3,4-c]pyrrol-1,4-dion-dibromid; 2,5-Bis[3-[4-(Dimethylamino)pyridinium]propyl]-3,6-diphenyl-pyrrolo[3,4-c]pyrrol-1,4-diondibromid; 2,5-Bis[2-[4-(Dimethylamino)pyridinium]ethyl]-3,6-diphenylpyrrolo-[3,4-c]pyrrol-1,4-dion-dibromid; 2,5-Bis[4-[4-(Dimethylamino)-pyridinium]butyl]-3,6-diphenyl-pyrrolo[3,4-c]-pyrrol-1,4-dion-dichlorid; 2,5-Bis[3-[4-(Dimethylamino)pyridinium]propyl]-3,6-diphenyl-pyrrolo[3,4-c]pyrrol-1,4-dion-dichlorid; 2,5-Bis[2-[4-(Dimethylamino)pyridinium]ethyl]-3,6-diphenyl-pyrrolo-[3,4-c]pyrrol-1,4-dion-dichlorid; 2,5-Bis(4-Trimethylammonium-butyl)-3,6-bis(4-chlor-phenyl)-pyrrolo[3,4-c]pyrrol-1,4-diondibromid; 2,5-Bis(3-Trimethylammonium-propyl)-3,6-bis(4-chlor-phenyl)-pyrrolo[3,4-c]pyrrol-1,4-dion-dibromid; 2,5-Bis(2-Trimethylammoniumethyl)-3,6-bis(4-chlor-phenyl)-pyrrolo-[3,4-c]pyrrol-1,4-dion-dibromid; 2,5-Bis(4-Trimethylammonium-butyl)-3,6-bis(4-chlor-phenyl)-pyrrolo[3,4-c]pyrrol-1,4-dion-dichlorid; 2,5-Bis(3-Trimethylammonium-propyl)-3,6-bis(4-chlor-phenyl)-pyrrolo[3,4-c]pyrrol-1,4-dion-dichlorid; 2,5-Bis(2-Trimethylammonium-ethyl)-3,6-bis(4-chlor-phenyl)-pyrrolo-[3,4-c]pyrrol-1,4-dion-dichlorid; 2,5-Bis[4-[4-(Dimethylamino)pyridinium]butyl]-3,6-bis(4-chlor-phenyl)-pyrrolo[3,4-c]pyrrol-1,4-dion-dibromid; 2,5-Bis[3-[4-(Dimethylamino)pyridinium]propyl]-3,6-bis(4-chlor-phenyl)-pyrrolo[3,4-c]pyrrol-1,4-dion-dibromid; 2,5-Bis[2-[4-(Dimethylamino)pyridinium]ethyl]-3,6-bis(4-chlor-phenyl)-pyrrolo[3,4-c]pyrrol-1,4-dion-dibromid; 2,5-Bis[4-[4-(Dimethylamino)pyridinium]butyl]-3,6-bis(4-chlorphenyl)-pyrrolo[3,4-c]-pyrrol-1,4-dion-dichlorid; 2,5-Bis[3-[4-(Dimethylamino)pyridinium]propyl]-3,6-bis(4-Chlor-phenyl)-pyrrolo[3,4-c]pyrrol-1,4-dion-dichlorid; 2,5-Bis[2-[4-(Dimethylamino)pyridinium]ethyl]-3,6-bis(4-chlor-phenyl)-pyrrolo[3,4-c]-pyrrol-1,4-dion-dichlorid; 2,5-Bis(4-Trimethylammonium-butyl)-3,6-bis(4-*tert*-butyl-phenyl)-pyrrolo[3,4-c]pyrrol-1,4-dion-dibromid; 2,5-Bis(3-Trimethylammonium-propyl)-3,6-bis(4-*tert*-butyl-phenyl)-pyrrolo[3,4-c]-pyrrol-1,4-dion-dibromid; 2,5-Bis(2-Trimethylammonium-ethyl)-3,6-bis(4-*tert*-butyl-phenyl)-pyrrolo-[3,4-c]pyrrol-1,4-dion-dibromid; 2,5-Bis(4-Trimethylammonium-butyl)-3,6-bis(4-*tert*-butyl-phenyl)-pyrrolo[3,4-c]pyrrol-1,4-dion-dichlorid; 2,5-Bis(3-Trimethylammonium-propyl)-3,6-bis(4-*tert*-butyl-phenyl)-pyrrolo[3,4-c]pyrrol-1,4-dion-dichlorid; 2,5-Bis(2-Trimethylammonium-ethyl)-3,6-bis(4-*tert*-butyl-phenyl)-pyrrolo[3,4-c]pyrrol-1,4-diondichlorid; 2,5-Bis[4-[4-(Dimethylamino)-pyridinium]butyl]-3,6-bis(4-*tert*-butyl-phenyl)-pyrrolo[3,4-c]pyrrol-1,4-dion-dibromid; 2,5-Bis[3-[4-(Dimethylamino)pyridinium]propyl]-3,6-bis(4-*tert*-butyl-phenyl)-pyrrolo[3,4-c]pyrrol-1,4-dion-dibromid; 2,5-Bis[2-[4-(Dimethylamino)-pyridinium]ethyl]-3,6-bis(4-*tert*-butyl-phenyl)-pyrrolo-[3,4-c]pyrrol-1,4-dion-dibromid; 2,5-Bis[4-[4-(Dimethylamino)pyridinium]butyl]-3,6-bis(4-*tert*-butyl-phenyl)-pyrrolo[3,4-c]-pyrrol-1,4-dion-dichlorid; 2,5-Bis[3-[4-(Dimethylamino)-pyridinium]propyl]-3,6-bis(4-*tert*-butyl-phenyl)-pyrrolo[3,4-c]pyrrol-1,4-diondichlorid; 2,5-Bis[2-[4-(Dimethylamino)pyridinium]ethyl]-3,6-bis(4-*tert*-butylphenyl)-pyrrolo-[3,4-c]pyrrol-1,4-dion-dichlorid; 2,5-Bis(4-Trimethylammonium-butyl)-3,6-bis(biphenyl)-pyrrolo[3,4-c]pyrrol-1,4-dion-dibromid; 2,5-Bis(3-Trimethylammonium-propyl)-3,6-bis(biphenyl)-pyrrolo[3,4-c]pyrrol-1,4-dion-dibromid; 2,5-Bis(2-Trimethylammonium-ethyl)-3,6-bis(biphenyl)-pyrrolo[3,4-c]pyrrol-1,4-dion-dibromid; 2,5-Bis(4-Trimethylammonium-butyl)-3,6-bis(biphenyl)-pyrrolo[3,4-c]pyrrol-1,4-dion-dichlorid; 2,5-Bis(3-Trimethylammonium-propyl)-3,6-bis(biphenyl)-pyrrolo[3,4-c]pyrrol-1,4-dion-dichlorid; 2,5-Bis(2-Trimethylammonium-ethyl)-3,6-bis(biphenyl)-pyrrolo[3,4-c]pyrrol-1,4-dion-dichlorid; 2,5-Bis[4-[4-(Dimethylamino)pyridinium]butyl]-3,6-bis(biphenyl)-pyrrolo[3,4-c]pyrrol-1,4-dion-dibromid; 2,5-Bis[3-[4-(Dimethylamino)pyridinium]propyl]-3,6-bis(biphenyl)-pyrrolo[3,4-c]pyrrol-1,4-dion-dibromid; 2,5-Bis[2-[4-(Dimethylamino)pyridinium]ethyl]-3,6-bis(biphenyl)-pyrrolo[3,4-c]pyrrol-1,4-dion-dibromid; 2,5-Bis[4-[4-(Dimethylamino)pyridinium]butyl]-3,6-bis(biphenyl)-pyrrolo[3,4-c]-pyrrol-1,4-dion-dichlorid; 2,5-Bis[3-[4-(Dimethylamino)pyridinium]propyl]-3,6-bis(biphenyl)-pyrrolo[3,4-c]pyrrol-1,4-dion-dichlorid; 2,5-Bis[2-[4-(Dimethylamino)pyridinium]ethyl]-3,6-bis(biphenyl)-pyrrolo-[3,4-c]pyrrol-1,4-dion-dichlorid; 2,5-Bis(4-Trimethylammonium-butyl)-3,6-bis(3-chlor-phenyl)-pyrrolo[3,4-c]pyrrol-1,4-dion-dibromid; 2,5-Bis(3-Trimethylammonium-propyl)-3,6-bis(3-chlorphenyl)-pyrrolo[3,4-c]pyrrol-1,4-dion-dibromid; 2,5-Bis(2-Trimethylammonium-ethyl)-3,6-bis(3-chlor-phenyl)-pyrrolo-[3,4-c]pyrrol-1,4-diondibromid; 2,5-Bis(4-Trimethylammonium-butyl)-3,6-bis(3-chlor-phenyl)-pyrrolo[3,4-c]pyrrol-1,4-dion-dichlorid; 2,5-Bis(3-Trimethylammoniumpropyl)-3,6-bis(3-chlor-phenyl)-pyrrolo[3,4-c]pyrrol-1,4-dion-dichlorid; 2,5-Bis(2-Trimethylammonium-ethyl)-3,6-bis(3-chlor-phenyl)-pyrrolo-[3,4-c]pyrrol-1,4-dion-dichlorid; 2,5-Bis[4-[4-(Dimethylamino)pyridinium]butyl]-3,6-bis(3-chlor-phenyl)-pyrrolo[3,4-c]pyrrol-1,4-dion-dibromid; 2,5-Bis[3-[4-(Dimethylamino)pyridinium]propyl]-3,6-bis(3-chlor-phenyl)-pyrrolo[3,4-c]pyrrol-1,4-dion-dibromid; 2,5-Bis[2-[4-(Dimethylamino)pyridinium]ethyl]-3,6-bis(3-chlor-phenyl)-pyrrolo[3,4-c]pyrrol-1,4-dion-dibromid; 2,5-Bis[4-[4-(Dimethylamino)pysidinium]butyl]-3,6-bis(3-chlor-phenyl)-pyrrolo[3,4-c]-pyrrol-1,4-dion-dichlorid; 2,5-Bis[3-[4-(Dimethylamino)pyridinium]propyl]-3,6-bis(3-chlor-phenyl)-pyrrolo[3,4-c]pyrrol-1,4-dion-dichlorid; 2,5-Bis[2-[4-(Dimethylamino)pyridinium]ethyl]-3,6-bis(3-chlor-phenyl)-pyrrolo[3,4-c]-pyrrol-1,4-dion-dichlorid; 2,5-Bis(4-Trimethylammonium-butyl)-3,6-bis(4-methyl-phenyl)-pyrrolo[3,4-c]pyrrol-1,4-dion-dibromid; 2,5-Bis(3-Trimethylammonium-propyl)-3,6-bis(4-methyl-phenyl)-pyrrolo[3,4-c]pyrrol-1,4-dion-dibromid; 2,5-Bis(2-Trimethylammonium-ethyl)-3,6-bis(4-methylphenyl)-pyrrolo[3,4-c]pyrrol-1,4-dion-dibromid; 2,5-Bis(4-Trimethylammonium-butyl)-3,6-bis(4-methyl-phenyl)-pyrrolo[3,4-c]pyrrol-1,4-diondichlorid; 2,5-Bis(3-Trimethylammonium-propyl)-3,6-bis(4-methyl-phenyl)-pyrrolo[3,4-c]pyrrol-1,4-dion-dichlorid; 2,5-Bis(2-Trimethylammoniumethyl)-3,6-bis(4-methyl-phenylrpyrrolo[3,4-c]pyrrol-1,4-dion-dichlorid; 2,5-Bis[4-[4-(Dimethylamino)pyridinium]butyl]-3,6-bis(4-methyl-phenyl)-pyrrolo[3,4-c]pyrrol-1,4-dion-dibromid; 2,5-Bis[3-[4-(Dimethylamino)-pyridinium]propyl]-3,6-bis(4-methyl-phenyl)-pyrrolo[3,4-c]pyrrol-1,4-diondibromid; 2,5-Bis[2-[4-(Dimethylamino)pyridinium]ethyl]-3,6-bis(4-methylphenyl)-pyrrolo[3,4-c]pyrrol-1,4-dion-dibromid; 2,5-Bis[4-[4-(Dimethylamino)pyridinium]butyl]-3,6-bis(4-methyl-phenyl)-pyrrolo[3,4-c]-pyrrol-1,4-dion-dichlorid; 2,5-Bis[3-[4-(Dimethylamino)pyridinium]propyl]-3,6-bis(4-methyl-phenyl)-pyrrolo[3,4-c]pyrrol-1,4-dion-dichlorid; 2,5-Bis[2-[4-(Dimethylamino)pyridinium]ethyl]-3,6-bis(4-methyl-phenyl)-pyrrolo[3,4-c]pyrrol-1,4-dion-dichlorid; 2,5-Bis(4-Trimethylammonium-butyl)-3,6-bis(3-methyl-phenyl)-pyrrolo[3,4-c]pyrrol-1,4-dion-dibromid; 2,5-Bis(3-Trimethylammonium-propyl)-3,6-bis(3-methyl-phenyl)-pyrrolo[3,4-c]pyrrol-1,4-dion-dibromid; 2,5-Bis(2-Trimethylammonium-ethyl)-3,6-bis(3-methylphenyl)-pyrrolo[3,4-c]pyrrol-1,4-dion-dibromid; 2,5-Bis(4-Trimethylammonium-butyl)-3,6-bis(3-methyl-phenyl)-pyrrolo[3,4-c]pyrrol-1,4-diondichlorid; 2,5-Bis(3-Trimethylammonium-propyl)-3,6-bis(3-methyl-phenyl)-pyrrolo[3,4-c]pyrrol-1,4-dion-dichlorid; 2,5-Bis(2-Trimethylammoniumethyl)-3,6-bis(3-methyl-phenyl)-pyrrolo[3,4-c]pyrrol-1,4-dion-dichlorid; 2,5-Bis[4-[4-(Dimethylamino)-pyridinium]butyl]-3,6-bis(3-methyl-phenyl)-pyrrolo[3,4-c]pyrrol-1,4-dion-dibromid; 2,5-Bis[3-[4-(Dimethylamino)-pyridinium]propyl]-3,6-bis(3-methyl-phenyl)-pyrrolo[3,4-c]pyrrol-1,4-diondibromid; 2,5-Bis[2-[4-(Dimethylamino)-pyridinium]ethyl]-3,6-bis(3-methylphenyl)-pyrrolo[3,4-c]pyrrol-1,4-dion-dibromid; 2,5-Bis[4-[4-(Dimethylamino)pyridinium]butyl]-3,6-bis(3-methyl-phenyl)-pyrrolo[3,4-c]-pyrrol-1,4-dion-dichlorid; 2,5-Bis[3-[4-(Dimethylamino)-pyridinium]propyl]-3,6-bis(3-methyl-phenyl)-pyrrolo[3,4-c]pyrrol-1,4-dion-dichlorid; 2,5-Bis[2-[4-(Dimethylamino)pyridinium]ethyl]-3,6-bis(3-methyl-phenyl)-pyrrolo[3,4-c]pyrrol-1,4-dion-dichlorid; 2,5-Bis(4-Trimethylammonium-butyl)-3,6-bis(4-cyano-phenyl)-pyrrolo[3,4-c]pyrrol-1,4-dion-dibromid; 2,5-Bis(3-Trimethylammonium-propyl)-3,6-bis(4-cyano-phenyl)-pyrrolo[3,4-c]pyrrol-1,4-dion-dibromid; 2,5-Bis(2-Trimethylammonium-ethyl)-3,6-bis(4-cyanophenyl)-pyrrolo[3,4-c]pyrrol-1,4-dion-dibromid; 2,5-Bis(4-Trimethylammonium-butyl)-3,6-bis(4-cyano-phenyl)-pyrrolo[3,4-c]pyrrol-1,4-diondichlorid; 2,5-Bis(3-Trimethylammonium-propyl)-3,6-bis(4-cyano-phenyl)-pyrrolo[3,4-c]pyrrol-1,4-dion-dichlorid; 2,5-Bis(2-Trimethylammoniumethyl)-3,6-bis(4-cyano-phenyl)-pyrrolo-[3,4-c]pyrrol-1,4-dion-dichlorid; 2,5-Bis[4-[4-(Dimethylamino)pyridinium]butyl]-3,6-bis(4-cyano-phenyl)-pyrrolo[3,4-c]pyrrol-1,4-dion-dibromid; 2,5-Bis[3-[4-(Dimethylamino)-pyridinium]propyl]-3,6-bis(4-cyano-phenyl)-pyrrolo[3,4-c]pyrrol-1,4-diondibromid; 2,5-Bis[2-[4-(Dimethylamino)pyridinium]ethyl]-3,6-bis(4-cyanophenyl)-pyrrolo[3,4-c]pyrrol-1,4-dion-dibromid; 2,5-Bis[4-[4-(Dimethylamino)pyridinium]butyl]-3,6-bis(4-cyano-phenyl)-pyrrolo[3,4-c]-pyrrol-1,4-dion-dichlorid; 2,5-Bis[3-[4-(Dimethylamino)pyridinium]propyl]-3,6-bis(4-cyano-phenyl)-pyrrolo[3,4-c]pyrrol-1,4-dion-dichlorid; 2,5-Bis[2-[4-(Dimethylamino)pyridinium]ethyl]-3,6-bis(4-cyano-phenyl)-pyrrolo[3,4-c]-pyrrol-1,4-dion-dichlorid; 2,5-Bis(4-Trimethylammonium-butyl)-3,6-bis(3-cyano-phenyl)-pyrrolo[3,4-c]pyrrol-1,4-dion-dibromid; 2,5-Bis(3-Trimethylammonium-propyl)-3,6-bis(3-cyano-phenyl)-pyrrolo[3,4-c]pyrrol-1,4-diondibromid; 2,5-Bis(2-Trimethylammonium-ethyl)-3,6-bis(3-cyano-phenyl)-pyrrolo[3,4-c]pyrrol-1,4-dion-dibromid; 2,5-Bis(4-Trimethyl-ammoniumbutyl)-3,6-bis(3-cyano-phenyl)-pyrrolo[3,4-c]pyrrol-1,4-dion-dichlorid; 2,5-Bis(3-Trimethylammonium-propyl)-3,6-bis(3-cyano-phenyl)-pyrrolo[3,4-c]pyrrol-1,4-dion-dichlorid; 2,5-Bis(2-Trimethylammonium-ethyl)-3,6-bis(3-cyano-phenyl)-pyrrolo-[3,4-c]pyrrol-1,4-dion-dichlorid; 2,5-Bis[4-[4-(Dimethylamino)pyridinium]butyl]-3,6-bis(3-cyano-phenyl)-pyrrolo[3,4-c]pyrrol-1,4-dion-dibromid; 2,5-Bis[3-[4-(Dimethylamino)-pyridinium]-propyl]-3,6-bis(3-cyano-phenyl)-pyrrolo[3,4-c]pyrrol-1,4-dion-dibromid; 2,5-Bis[2-[4-(Dimethylamino)pyridinium]ethyl]-3,6-bis(3-cyano-phenyl)-pyrrolo[3,4-c]pyrrol-1,4-dion-dibromid; 2,5-Bis[4-[4-(Dimethylamino)-pyridinium]butyl]-3,6-bis(3-cyano-phenyl)-pyrrolo[3,4-c]-pyrrol-1,4-diondichlorid; 2,5-Bis[3-[4-(Dimethylamino)pyridinium]propyl]-3,6-bis(3-cyanophenyl)-pyrrolo[3,4-c]pyrrol-1,4-dion-dichlorid; 2,5-Bis[2-[4-(Dimethylamino)pyridinium]ethyl]-3,6-bis(3-cyano-phenyl)-pyrrolo[3,4-c]-pyrrol-1,4-dion-dichlorid; 2,5-Bis(4-Trimethylammonium-butyl)-3,6-bis(4-dimethylaminophenyl)-pyrrolo[3,4-c]pyrrol-1,4-dion-dibromid; 2,5-Bis(3-Trimethylammonium-propyl)-3,6-bis(4-dimethylamino-phenyl)-pyrrolo[3,4-c]pyrrol-1,4-dion-dibromid; 2,5-Bis(2-Trimethylammonium-ethyl)-3,6-bis(4-dimethylamino-phenyl)-pyrrolo[3,4-c]pyrrol-1,4-dion-dibromid; 2,5-Bis(4-Trimethylammonium-butyl)-3,6-bis(4-dimethylamino-phenyl)-pyrrolo[3,4-c]-pyrrol-1,4-dion-dichlorid; 2,5-Bis(3-Trimethylammonium-propyl)-3,6-bis(4-dimethylamino-phenyl)-pyrrolo[3,4-c]pyrrol-1,4-dion-dichlorid; 2,5-Bis(2-Trimethylammonium-ethyl)-3,6-bis(4-dimethylamino-phenyl)-pyrrolo-[3,4-c]pyrrol-1,4-dion-dichlorid; 2,5-Bis[4-[4-(Dimethylamino)pyridinium]butyl]-3,6-bis(4-dimethylamino-phenyl)-pyrrolo[3,4-c]pyrrol-1,4-dion-dibromid; 2,5-Bis[3-[4-(Dimethylamino)pyridinium]propyl]-3,6-bis(4-dimethylaminophenyl)-pyrrolo[3,4-c]pyrrol-1,4-dion-dibromid; 2,5-Bis[2-[4-(Dimethylamino)pyridinium]ethyl]-,6-bis(4-dimethylamino-phenyl)-pyrrolo[3,4-c]-pyrrol-1,4-dion-dibromid; 2,5-Bis[4-[4-(Dimethylamino)pyridinium]butyl]-3,6-bis(4-dimethylamino-phenyl)-pyrrolo[3,4-c]-pyrrol-1,4-dion-dichlorid; 2,5-Bis[3-[4-(Dimethylamino)pyridinium]propyl]-3,6-bis(4-dimethylaminophenyl)-pyrrolo[3,4-c]pyrrol-1,4-dion-dichlorid; 2,5-Bis[2-[4-(Dimethylamino)pyridinium]ethyl]-3,6-bis(4-dimethylaminophenyl)-pyrrolo[3,4-c]pyrrol-1,4-dion-dichlorid; 2,5-Bis[4-[N-(Methylimidazolium)butyl]]-3,6-diphenyl-pyrrolo[3,4-c]pyrrol-1,4-dion-dibromid; 2,5-Bis[3-[N-(Methylimidazolium)propyl]]-3,6-diphenyl-pyrrolo[3,4-c]pyrrol-1,4-dion-dibromid; 2,5-Bis[2-[N-(Methylimidazolium)ethyl]]-3,6-diphenyl-pyrrolo-[3,4-c]pyrrol-1,4-dion-dibromid; 2,5-Bis[4-[N-(Methylimidazolium)butyl]]-3,6-diphenylpyrrolo[3,4-c]-pyrrol-1,4-dion-dichlorid; 2,5-Bis[3-[N-(Methylimidazolium)-propyl]]-3,6-diphenyl-pyrrolo[3,4-c]pyrrol-1,4-dion-dichlorid; 2,5-Bis[2-[N-(Methylimidazolium)ethyl)]-3,6-diphenyl-pyrrolo-[3,4-c]pyrrol-1,4-diondichlorid; 2,5-Bis[4-[N-(Methylimidazolium)butyl]]-3,6-bis(4-*tert*-butylphenyl)-pyrrolo[3,4-c]pyrrol-1,4-dion-dibromid; 2,5-Bis[3-[N-(Methylimidazolium)propyl]]-3,6-bis(4-*tert*-butyl-phenyl)-pyrrolo-[3,4-c]pyrrol-1,4-dion-dibromid; 2,5-Bis[2-[N-(Methylimidazolium)ethyl]]-3,6-bis(4-*tert*-butylphenyl)-pyrrolo-[3,4-c]pyrrol-1,4-dion-dibromid; 2,5-Bis[4-[N-(Methylimidazolium)butyl]]-3,6-bis(4-*tert*-butyl-phenyl)-pyrrolo[3,4-c]-pyrrol-1,4-dion-dichlorid; 2,5-Bis[3-[N-(Methylimidazolium)-propyl]]-3,6-bis(4-*tert*-butyl-phenyl)-pyrrolo[3,4-c]pyrrol-1,4-dion-dichlorid; 2,5-Bis[2-[ N-(Methylimidazolium)ethyl]]-3,6-bis(4-*tert*-butyl-phenyl)-pyrrolo-[3,4-c]pyrrol-1,4-dion-dichlorid; 2,5-Bis[4-[N-(Methylimidazolium)butyl]]-3,6-bis(4-chlorophenyl)-pyrrolo[3,4-c]pyrrol-1,4-dion-dibromid; 2,5-Bis[3-[N-(Methylimidazolium)propyl]]-3,6-bis(4-chloro-phenyl)-pyrrolo-[3,4-c]pyrrol-1,4-dion-dibromid; 2,5-Bis[2-[N-(Methylimidazolium)ethyl]]-3,6-bis(4-chlorophenyl)-pyrrolo-[3,4-c]pyrrol-1,4-dion-dibromid; 2,5-Bis[4-[N-(Methylimidazolium)butyl]]-3,6-bis(4-chloro-phenyl)-pyrrolo[3,4-c]-pyrrol-1,4-diondichlorid; 2,5-Bis[3-[N-(Methylimidazolium)-propyl]]-3,6-bis(4-chlorophenyl)-pyrrolo[3,4-c]pyrrol-1,4-dion-dichlorid; 2,5-Bis[2-[N-(Methylimidazolium)ethyl]]-3,6-bis(4-chloro-phenyl)-pyrrolo-[3,4-c]pyrrol-1,4-diondichlorid;2,5-Bis[4-[N-(Methylimidazolium)butyl]]-3,6-bis(4-methyl-phenyl)-pyrrolo[3,4-c]pyrrol-1,4-dion-dibromid; 2,5-Bis[3-[N-(Methyl-imidazolium)-propyl]]-3,6-bis(4-methyl-phenyl)-pyrrolo-[3,4-c]pyrrol-1,4-dion-dibromid; 2,5-Bis[2-[N-(Methylimidazolium)ethyl]]-3,6-bis(4-methyl-phenyl)-pyrrolo-[3,4-c]pyrrol-1,4-dion-dibromid; 2,5-Bis[4-[N-(Methyl-imidazolium]butyl]]-3,6-bis(4-methyl-phenyl)-pyrrolo[3,4-c]-pyrrol-1,4-dion-dichlorid; 2,5-Bis[3-[N-(Methylimidazolium)-propyl]]-3,6-bis(4-methyl-phenyl)-pyrrolo[3,4-c]pyrrol-1,4-dion-dichlorid; 2,5-Bis[2-[N-(Methyl-imidazolium)ethyl]]-3,6-bis(4-methyl-phenyl)-pyrrolo-[3,4-c]pyrrol-1,4-dion-dichlorid; 2,5-Bis[4-[N-(Methylimidazolium)butyl]]-3,6-bis(3-cyano-phenyl)-pyrrolo[3,4-c]pyrrol-1,4-dion-dibromid; 2,5-Bis[3-[N-(Methyl-imidazolium)-propyl]]-3,6-bis(3-cyano-phenyl)-pyrrolo-[3,4-c]pyrrol-1,4-dion-dibromid; 2,5-Bis[2-[N-(Methylimidazolium)ethyl]]-3,6-bis(3-cyano-phenyl)-pyrrolo-[3,4-c]pyrrol-1,4-dion-dibromid; 2,5-Bis[4-[N-(Methyl-imidazolium)butyl]]-3,6-bis(3-cyano-phenyl)-pyrrolo[3,4-c]-pyrrol-1,4-dion-dichlorid; 2,5-Bis[3-[N-(Methylimidazolium)-propyl]]-3,6-bis(3-cyano-phenyl)-pyrrolo[3,4-c]pyrrol-1,4-dion-dichlorid; 2,5-Bis[2-[ N-(Methyl-imidazolium)ethyl]]-3,6-bis(3-cyano-phenyl)-pyrrolo-[3,4-c]pyrrol-1,4-dion-dichlorid; 2,5-Bis[4-[N-(Methylimidazolium)butyl]]-3,6-bis(4-cyano-phenyl)-pyrrolo[3,4-c]pyrrol-1,4-dion-dibromid; 2,5-Bis[3-[N-(Methyl-imidazolium)-propyl]]-3,6-bis(4-cyano-phenyl)-pyrrolo-[3,4-c]pyrrol-1,4-dion-dibramid; 2,5-Bis[2-[N-(Methylimidazolium)ethyl]]-3,6-bis(4-cyano-phenyl)-pyrrolo-[3,4-c]pyrrol-1,4-dion-dibromid; 2,5-Bis[4-[N-(Methyl-imidazolium)butyl]]-3,6-bis(4-cyano-phenyl)-pyrrolo[3,4-c]-pyrrol-1,4-dion-dichlorid; 2,5-Bis[3-[N-(Methylimidazolium)-propyl]]-3,6-bis(4-cyano-phenyl)-pyrrolo[3,4-c]pyrrol-1,4-dion-dichlorid; 2,5-Bis[2-[ N-(Methylimidazolium)-ethyl]]-3,6-bis(4-cyano-phenyl)-pyrrolo-[3,4-c]pyrrol-1,4-dion-dichlorid; 2,5-Bis[4-[N-(Methylimidazolium)butyl]]-3,6-bis(3,4-dichloro-phenyl)-pyrrolo[3,4-c]pyrrol-1,4-dion-dibromid; 2,5-Bis[3-[N-(Methyl-imidazolium)-propyl]]-3,6-bis(3,4-dichloro-phenyl)-pyrrolo-[3,4-c]pyrrol-1,4-diondibromid; 2,5-Bis[2-[N-(Methylimidazolium)ethyl]]-3,6-bis(3,4-dichlorophenyl)-pyrrolo-[3,4-c]pyrrol-1,4-dion-dibromid; 2,5-Bis[4-[N-(Methylimidazolium)butyl]]-3,6-bis(3,4-dichloro-phenyl)-pyrrolo[3,4-c]-pyrrol-1,4-dion-dichlorid; 2,5-Bis[3-[N-(Methylimidazolium)-propyl]]-3,6-bis(3,4-dichloro-phenyl)-pyrrolo[3,4-c]pyrrol-1,4-dion-dichlorid; 2,5-Bis[2-[N-(Methylimidazolium)ethyl]]-3,6-bis(3,4-dichloro-phenyl)-pyrrolo-[3,4-c]pyrrol-1,4-dion-dichlorid; 2,5-Bis[4-[N-(Methylimidazolium)butyl]]- 3,6-bis(biphenyl)-pyrrolo[3,4-c]pyrrol-1,4-dion-dibromid; 2,5-Bis[3-[N-(Methylimidazolium)propyl]]-3,6-bis(biphenyl)-pyrrolo-[3,4-c]pyrrol-1,4-diondibromid; 2,5-Bis[2-[N-(Methylimidazolium)ethyl]]- 3,6-bis(biphenyl)-pyrrolo-[3,4-c]pyrrol-1,4-dion-dibromid; 2,5-Bis[4-[N-(Methyl-imidazolium)-butyl]]- 3,6-bis(biphenyl)-pyrrolo[3,4-c]-pyrrol-1,4-dion-dichlorid; 2,5-Bis[3-[N-(Methylimidazolium)-propyl]]- 3,6-bis(biphenyl)-pyrrolo[3,4-c]pyrrol-1,4-dion-dichlorid; 2,5-Bis[2-[ N-(Methylimidazolium)ethyl]]- 3,6-bis(biphenyl)-pyrrolo-[3,4-c]pyrrol-1,4-dion-dichlorid; 2, 5-Bis(4-Trimethylammonium-butyl)-3,6-bis(4-methoxy-phenyl)-pyrrolo[3,4-c]pyrrol-1,4-dion-dibromid; 2,5-Bis(3-Trimethyl-ammoniumpropyl)-3,6-bis(4-methoxy -phenyl)-pyrrolo[3,4-c]pyrrol-1,4-dion-dibromid; 2,5-Bis(2-Trimethylammonium-ethyl)-3,6-bis(4-methoxy-phenyl)-pyrrolo[3,4-c]pyrrol-1,4-dion-dibromid; 2,5-Bis(4-Trimethyl-ammoniumbutyl)-3,6-bis(4-methoxy-phenyl)-pyrrolo[3,4-c]pyrrol-1,4-dion-dichlorid; 2,5-Bis(3-Trimethylammonium-propyl)-3,6-bis(4-methoxy-phenyl)-pyrrolo[3,4-c]pyrrol-1,4-dion-dichlorid; 2,5-Bis(2-Trimethylammoniumethyl)-3,6-bis(4-methoxy-phenyl)-pyrrolo-[3,4-c]pyrrol-1,4-dion-dichlorid; 2,5-Bis[4-[4-(Dimethylamino)pyridinium]butyl]-3,6-bis(4-methoxy-phenyl)-pyrrolo[3,4-c]pyrrol-1,4-dion-dibromid; 2,5-Bis[3-[4-(Dimethylamino)-pyridinium]-propyl]-3,6-bis(4-methoxy-phenyl)-pyrrolo[3,4-c]pyrrol-1,4-dion-dibromid; 2,5-Bis[2-[4-(Dimethylamino)pyridinium]ethyl]-3,6-bis(4-methoxy-phenyl)-pyrrolo[3,4-c]pyrrol-1,4-dion-dibromid; 2,5-Bis[4-[4-(Dimethylamino)-pyridinium]butyl]-3,6-bis(4-methoxy-phenyl)-pyrrolo[3,4-c]-pyrrol-1,4-dion-dichlorid; 2,5-Bis[3-[4(Dimethylamino)pyridinium]propyl]-3,6-bis(4-methoxy-phenyl)-pyrrolo[3,4-c]pyrrol-1,4-dion-dichlorid; 2,5-Bis[2-[4-(Dimethyl-amino)pyridinium]ethyl]-3,6-bis(4-methoxy-phenyl)-pyrrolo[3,4-c]-pyrrol-1,4-dion-dichlorid; 2,5-Bis(4-Trimethylammoniumbutyl)-3,6-bis(3-methoxy-phenyl)-pyrrolo[3,4-c]pyrrol-1,4-dion-dibromid; 2,5-Bis(3-Trimethyl-ammonium-propyl)-3,6-bis(3-methoxy -phenyl)-pyrrolo[3,4-c]pyrrol-1,4-dion-dibromid; 2,5-Bis(2-Trimethylammoniumethyl)-3,6-bis(3-methoxy-phenyl)-pyrrolo[3,4-c]pyrrol-1,4-dion-dibromid; 2,5-Bis(4-Trimethyl-ammonium-butyl)-3,6-bis(3-methoxy-phenyl)-pyrrolo[3,4-c]pyrrol-1,4-dion-dichlorid; 2,5-Bis(3-Trimethylammoniumpropyl)-3,6-bis(3-methoxy-phenyl)-pyrrolo[3,4-c]pyrrol-1,4-dion-dichlorid; 2,5-Bis(2-Trimethylammonium-ethyl)-3,6-bis(3-methoxy-phenyl)-pyrrolo-[3,4-c]pyrrol-1,4-dion-dichlorid; 2,5-Bis[4-[4-(Dimethylamino)pyridinium]-butyl]-3,6-bis(3-methoxy-phenyl)-pyrrolo[3,4-c]pyrrol-1,4-dion-dibromid; 2,5-Bis[3-[4-(Dimethylamino)-pyridinium]-propyl]-3,6-bis(3-methoxyphenyl)-pyrrolo[3,4-c]pyrrol-1,4-dion-dibromid; 2,5-Bis[2-[4-(Dimethytamino)pyridinium)ethyl]-3,6-bis(3-methoxy-phenyl)-pyrrolo[3,4-c]pyrrol-1,4-dion-dibromid; 2,5-Bis[4-[4-(Dimethylamino)-pyridinium]butyl]-3,6-bis(3-methoxy-phenyl)-pyrrolo[3,4-c]-pyrrol-1,4-dion-dichlorid; 2,5-Bis[3-[4(Dimethylamino)pyridinium]propyl]-3,6-bis(3-methoxy-phenyl)-pyrrolo[3,4-c]pyrrol-1,4-dion-dichlorid; 2,5-Bis[2-[4-(Dimethylamino)-pyridinium]ethyl]-3,6-bis(3-methoxy-phenyl)-pyrrolo[3,4-c]-pyrrol-1,4-diondichlorid; 2,5-Bis(4-Trimethylammonium-butyl)-3,6-bis(3,4-dichlorophenyl)-pyrrolo[3,4-c]pyrrol-1,4-dion-dibromid; 2,5-Bis(3-Trimethylammonium-propyl)-3,6-bis(3,4-dichloro-phenyl)-pyrrolo[3,4-c]pyrrol-1,4-dion-dibromid; 2,5-Bis(2-Trimethylammonium-ethyl)-3,6-bis(3,4-dichlorophenyl)-pyrrolo[3,4-c]pyrrol-1,4-dion-dibromid; 2,5-Bis(4-Trimethylammonium-butyl)-3,6-bis(3,4-dichloro-phenyl)-pyrrolo[3,4-c]pyrrol-1,4-dion-dichlorid; 2,5-Bis(3-Trimethylammonium-propyl)-3,6-bis(3,4-dichlorophenyl)-pyrrolo[3,4-c]pyrrol-1,4-dion-dichlorid; 2,5-Bis(2-Trimethylammonium-ethyl)-3,6-bis(3,4-dichloro-phenyl)-pyrrolo-[3,4-c]pyrrol-1,4-dion-dichlorid; 2,5-Bis[4-[4-(Dimethylamino)pyridinium]-butyl]-3,6-bis(3,4-dichloro-phenyl)-pyrrolo[3,4-c]pyrrol-1,4-dion-dibromid; 2,5-Bis[3-[4-(Dimethylamino)-pyridinium]-propyl]-3,6-bis(3,4-dichlor-phenyl)-pyrrolo[3,4-c]pyrrol-1,4-dion-dibromid; 2,5-Bis[2-[4-(Dimethylamino)-pyridinium]ethyl]-3,6-bis(3,4-dichloro-phenyl)-pyrrolo[3,4-c]pyrrol-1,4-diondibromid; 2,5-Bis[4-[4-(Dimethylamino)-pyridinium]butyl]-3,6-bis(3,4-dichloro-phenyl)-pyrrolo[3,4-c]-pyrrol-1,4-dion-dichlorid; 2,5-Bis[3-[4(Dimethylamino)pyridinium]propyl]-3,6-bis(3,4-dichloro-phenyl)-pyrrolo[3,4-c]pyrrol-1,4-dion-dichlorid; 2,5-Bis[2-[4-(Dimethylamino)-pyridinium]ethyl]-3,6-bis(3,4-dichloro-phenyl)-pyrrolo[3,4-c]-pyrrol-1,4-diondichlorid.

Unter den vorgenannten Verbindungen der Formel (I) sind die folgenden Verbindungen bevorzugt: 2,5-Bis(4-Trimethylammonium-butyl)-3,6-diphenyl-pyrrolo[3,4-c]pyrrol-1,4-dion-dibromid; 2,5-Bis(3-Trimethylammonium-propyl)-3,6-diphenyl-pyrrolo[3,4-c]pyrrol-1,4-dion-dibromid; 2,5-Bis(2-Trimethylammonium-ethyl)-3,6-diphenyl-pyrrolo-[3,4-c]pyrrol-1,4-dion-dibromid; 2,5-Bis(4-Trimethylammonium-butyl)-3,6-diphenylpyrrolo[3,4-c]pyrrol-1,4-dion-dichlorid; 2,5-Bis(3-Trimethylammoniumpropyl)-3,6-diphenyl-pyrrolo[3,4-c]pyrrol-1,4-dion-dichlorid; 2,5-Bis(2-Trimethylammonium-ethyl)-3,6-diphenyl-pyrrolo-[3,4-c]pyrrol-1,4-diondichlorid; 2,5-Bis[4-[4-(Dimethylamino)pyridinium]butyl]-3,6-diphenylpyrrolo[3,4-c]pyrrol-1,4-dion-dibromid; 2,5-Bis[3-[4-(Dimethylamino)-pyridinium]propyl]-3,6-diphenyl-pyrrolo[3,4-c]pyrrol-1,4-dion-dibromid; 2,5-Bis[2-[4-(Dimethylamino)pyridinium]ethyl]-3,6-diphenyl-pyrrolo[3,4-c]-pyrrol-1,4-dion-dibromid; 2,5-Bis[4-[4-(Dimethylamino)pyridinium]butyl]-3,6-diphenyl-pyrrolo[3,4-c]-pyrrol-1,4-dion-dichlorid; 2,5-Bis[3-[4-(Dimethylamino)pyridinium]propyl]-3,6-diphenyl-pyrrolo[3,4-c]pyrrol-1,4-dion-dichlorid; 2,5-Bis[2-[4-(Dimethylamino)pyridinium]ethyl]-3,6-diphenylpyrrolo[3,4-c]pyrrol-1,4-dion-dichlorid; 2,5-Bis(4-Trimethylammoniumbutyl)-3,6-bis(4-chlorphenyl)-pyrrolo[3,4-c]pyrrol-1,4-dion-dibromid; 2,5-Bis(3-Trimethylammonium-propyl)-3,6-bis(4-chlor-phenyl)-pyrrolo[3,4-c]pyrrol-1,4-dion-dibromid; 2,5-Bis(2-Trimethylammonium-ethyl)-3,6-bis(4-chlor-phenyl)-pyrrolo-[3,4-c]pyrrol-1,4-dion-dibromid; 2,5-Bis(4-Trimethylammonium-butyl)-3,6-bis(4-chlor-phenyl)-pyrrolo[3,4-c]pyrrol-1,4-dion-dichlorid; 2,5-Bis(3-Trimethylammonium-propyl)-3,6-bis(4-chlorphenyl)-pyrrolo[3,4-c]pyrrol-1,4-dion-dichlorid; 2,5-Bis(2-Trimethylammonium-ethyl)3,6-bis(4-chlor-phenyl)-pyrrolo-[3,4-c]pyrrol-1,4-diondichlorid; 2,5-Bis[4-[4-(Dimethylamino)pyridinium]butyl]-3,6-bis(4-chlorphenyl)-pyrrolo[3,4-c]pyrrol-1,4-dion-dibromid; 2,5-Bis[3-[4-(Dimethylamino)pyridinium]propyl]-3,6-bis(4-chlor-phenyl)-pyrrolo[3,4-c]pyrrol-1,4-dion-dibromid; 2,5-Bis[2-[4-(Dimethylamino)pyridinium]ethyl]-3,6-bis(4-chlor-phenyl)-pyrrolo-[3,4-c]pyrrol-1,4-dion-dibromid; 2,5-Bis[4-[4-(Dimethylamino)pyridinium]butyl]-3,6-bis(4-chlor-phenyl)-pyrrolo[3,4-c]-pyrrol-1,4-dion-dichlorid; 2,5-Bis[3-[4-(Dimethylamino)pyridinium]propyl]-3,6-bis(4-chlor-phenyl)-pyrrolo[3,4-c]pyrrol-1,4-dion-dichlorid; 2,5-Bis[2-[4-(Dimethylamino)pyridinium]ethyl]-3,6-bis(4-chlor-phenyl)-pyrrolo-[3,4-c]-pyrrol-1,4-dion-dichlorid; 2,5-Bis(4-Trimethylammonium-butyl)-3,6-bis(4-methyl-phenyl)-pyrrolo[3,4-c]pyrrol-1,4-dion-dibromid; 2,5-Bis(3-Trimethylammonium-propyl)-3,6-bis(4-methyl-phenyl)-pyrrolo[3,4-c]pyrrol-1,4-diondibromid; 2,5-Bis(2-Trimethylammonium-ethyl)-3,6-bis(4-methyl-phenyl)-pyrrolo[3,4-c]pyrrol-1,4-dion-dibromid; 2,5-Bis(4-Trimethyl-ammoniumbutyl)-3,6-bis(4-methyl-phenyl)-pyrrolo[3,4-c]pyrrol-1,4-dion-dichlorid; 2,5-Bis(3-Trimethylammonium-propyl)-3,6-bis(4-methyl-phenyl)-pyrrolo[3,4-c]pyrrol-1,4-dion-dichlorid; 2,5-Bis(2-Trimethylammoniumethyl)-3,6-bis(4-methyl-phenyl)-pyrrolo[3,4-c]pyrrol-1,4-dion-dichlorid; 2,5-Bis[4-(4-(Dimethylamino)pyridinium]butyl]-3,6-bis(4-methyl-phenyl)-pyrrolo[3,4-c]pyrrol-1,4-dion-dibromid; 2,5-Bis[3-[4-(Dimethylamino)-pyridinium]propyl]-3,6-bis(4-methyl-phenyl)-pyrrolo[3,4-c]pyrrol-1,4-diondibromid; 2,5-Bis[2-[4-(Dimethylamino)pyridinium]ethyl]-3,6-bis(4-methylphenyl)-pyrrolo[3,4-c]pyrrol-1,4-dion-dibromid; 2,5-Bis[4-[4-(Dimethylamino)pyridinium]butyl]-3,6-bis(4-methyl-phenyl)-pyrrolo[3,4-c]-pyrrol-1,4-dion-dichlorid; 2,5-Bis[3-[4-(Dimethylamino)pyridinium]propyl]-3,6-bis(4-methyl-phenyl)-pyrrolo[3,4-c]pyrrol-1,4-dion-dichlorid; 2,5-Bis[2-[4-(Dimethylamino)pyridinium]ethyl]-3,6-bis(4-methyl-phenyl)-pyrrolo[3,4-c]pyrrol-1,4-dion-dichlorid; 2,5-Bis(4-Trimethylammonium-butyl)-3,6-bis(4-*tert*-butyl-phenyl)-pyrrolo[3,4-c]pyrrol-1,4-dion-dibromid; 2,5-Bis(3-Trimethylammonium-propyl)-3,6-bis(4-*tert*-butyl-phenyl)-pyrrolo[3,4-c]pyrrol-1,4-dion-dibromid; 2,5-Bis(2-Trimethylammonium-ethyl)-3,6-bis(4-*tert*-butyl-phenyl)-pyrrolo[3,4-c]pyrrol-1,4-dion-dibromid; 2,5-Bis(4-Trimethylammonium-butyl)-3,6-bis(4-*tert*-butyl-phenyl)-pyrrolo[3,4-c]pyrrol-1,4-dion-dichlorid; 2,5-Bis(3-Trimethylammonium-propyl)-3,6-bis(4-*tert*-butyl-phenyl)-pyrrolo[3,4-c]pyrrol-1,4-dion-dichlorid; 2,5-Bis(2-Trimethylammonium-ethyl)-3,6-bis(4-*tert*-butyl-phenyl)-pyrrolo[3,4-c]pyrrol-1,4-diondichlorid; 2,5-Bis[4-[4-(Dimethylamino)pyridinium]butyl]-3,6-bis(4-*tert*-butylphenyl)-pyrrolo[3,4-c]pyrrol-1,4-dion-dibromid; 2,5-Bis[3-[4-(Dimethylamino)pyridinium]propyl]-3,6-bis(4-*tert*-butyl-phenyl)-pyrrolo[3,4-c]pyrrol-1,4-dion-dibromid; 2,5-Bis[2-[4-(Dimethylamino)pyridinium]ethyl]-3,6-bis(4-*tert*-butyl-phenyl)-pyrrolo[3,4-c]pyrrol-1,4-dion-dibromid; 2,5-Bis[4-[4-(Dimethylamino)pyridinium]butyl]-3,6-bis(4-*tert*-butyl-phenyl)-pyrrolo[3,4-c]-pyrrol-1,4-dion-dichlorid; 2,5-Bis[3-[4-(Dimethylamino)-pyridinium]-propyl]-3,6-bis(4-*tert*-butyl-phenyl)-pyrrolo[3,4-c]pyrrol-1,4-dion-dichlorid; 2,5-Bis[2-[4-(Dimethylamino)pyridinium]ethyl]-3,6-bis(4-*tert*-butyl-phenyl)-pyrrolo[3,4-c]pyrrol-1,4-dion-dichlorid; 2,5-Bis(4-Trimethyl-ammoniumbutyl)-3,6-bis(biphenyl)-pyrrolo[3,4-c]pyrrol-1,4-dion-dibromid; 2,5-Bis(3-Trimethylammonium-propyl)-3,6-bis(biphenyl)-pyrrolo[3,4-c]-pyrrol-1,4-dion-dibromid; 2,5-Bis(2-Trimethylammonium-ethyl)-3,6-bis(biphenyl)-pyrrolo[3,4-c]pyrrol-1,4-dion-dibromid; 2,5-Bis(4-Trimethylammoniumbutyl)-3,6-bis(biphenyl)-pyrrolo[3,4-c]pyrrol-1,4-dion-dichlorid; 2,5-Bis{3-Trimethylammonium-propyl)-3,6-bis(biphenyl)-pyrrolo[3,4-c]pyrrol-1,4-dion-dichlorid; 2,5-Bis(2-Trimethylammonium-ethyl)-3,6-bis(biphenyl)-pyrrolo-[3,4-c]pyrrol-1,4-dion-dichlorid; 2,5-Bis[4-[4-(Dimethylamino)-pyridinium]butyl]-3,6-bis(biphenyl)-pyrrolo[3,4-c]pyrrol-1,4-dion-dibromid; 2,5-Bis[3-[4-(Dimethylamino)pyridinium]propyl]-3,6-bis(biphenyl)-pyrrolo[3,4-c]pyrrol-1,4-dion-dibromid; 2,5-Bis[2-[4-(Dimethylamino)-pyridinium]ethyl]-3,6-bis(biphenyl)-pyrrolo[3,4-c]pyrrol-1,4-dion-dibromid; 2,5-Bis[4-[4-(Dimethylamino)pyridinium]butyl]-3,6-bis(biphenyl)-pyrrolo[3,4-c]-pyrrol-1,4-dion-dichlorid; 2,5-Bis[3-[4-(Dimethylamino)-pyridinium]propyl]-3,6-bis(biphenyl)-pyrrolo[3,4-c]pyrrol-1,4-dion-dichlorid; 2,5-Bis[2-[4-(Dimethylamino)pyridinium]ethyl]-3,6-bis(biphenyl)-pyrrolo[3,4-c]pyrrol-1,4-dion-dichlorid; 2,5-Bis(4-Trimethyl-ammoniumbutyl)-3,6-bis(4-dimethylamino-phenyl)-pyrrolo[3,4-c]pyrrol-1,4-diondibromid; 2,5-Bis(3-Trimethylammonium-propyl)-3,6-bis(4-dimethylaminophenyl)-pyrrolo[3,4-c]pyrrol-1,4-dion-dibromid; 2,5-Bis(2-Trimethylammonium-ethyl)-3,6-bis(4-dimethylamino-phenyl)-pyrrolo-[3,4-c]-pyrrol-1,4-dion-dibromid; 2,5-Bis(4-Trimethylammonium-butyl)-3,6-bis(4-dimethylamino-phenyl)-pyrrolo[3,4-c]pyrrol-1,4-dion-dichlorid; 2,5-Bis(3-Trimethylammonium-propyl)-3,6-bis(4-dimethylamino-phenyl)-pyrrolo[3,4-c]pyrrol-1,4-dion-dichlorid; 2,5-Bis(2-Trimethylammonium-ethyl)-3,6-bis(4-dimethylamino-phenyl)-pyrrolo[3,4-c]pyrrol-1,4-dion-dichlorid; 2,5-Bis[4-[4-(Dimethylamino)pyridinium]butyl]-3,6-bis(4-dimethylamino-phenyl)-pyrrolo[3,4-c]pyrrol-1,4-dion-dibromid; 2,5-Bis[3-[4-(Dimethylamino)-pyridinium]propyl]-3,6-bis(4-dimethylaminophenyl)-pyrrolo[3,4-c]pyrrol-1,4-dion-dibromid; 2,5-Bis[2-[4-(Dimethylamino)pyridinium]ethyl]-3,6-bis(4-dimethylaminophenyl)-pyrrolo[3,4-c]pyrrol-1,4-dion-dibromid; 2,5-Bis[4-[4-(Dimethylamino)pyridinium]butyl]-3,6-bis(4-dimethylamino-phenyl)-pyrrolo[3,4-c]-pyrrol-1,4-dion-dichlorid; 2,5-Bis[3-[4-(Dimethylamino)-pyridinium]propyl]-3,6-bis(4-dimethylamino-phenyl)-pyrrolo[3,4-c]pyrrol-1,4-dion-dichlorid; 2,5-Bis[2-[4-(Dimethylamino)pyridinium]ethyl]-3,6-bis(4-dimethylaminophenyl)-pyrrolo[3,4-c]pyrrol-1,4-dion-dichlorid; 2,5-Bis[4-[N-(Methylimidazolium)butyl]]-3,6-diphenyl-pyrrolo[3,4-c]pyrrol-1,4-diondibromid; 2,5-Bis[3-[N-(Methyl-imidazolium)propyl]]-3,6-diphenyl-pyrrolo-[3,4-c]pyrrol-1,4-dion-dibromid; 2,5-Bis[2-[N-(Methylimidazolium)ethyl]]-3,6-diphenyl-pyrrolo-[3,4-c]pyrrol-1,4-dion-dibromid; 2,5-Bis[4-[N-(Methylimidazolium)butyl]]-3,6-diphenyl-pyrrolo[3,4-c]-pyrrol-1,4-dion-dichlorid; 2,5-Bis[3-[N-(Methylimidazolium)-propyl]]-3,6-diphenyl-pyrrolo[3,4-c]pyrrol-1,4-dion-dichlorid; 2,5-Bis[2-[ N-(Methylimidazolium)ethyl]]-3,6-diphenylpyrrolo-[3,4-c]pyrrol-1,4-dion-dichlorid; 2,5-Bis[4-[N-(Methylimidazolium)-butyl]]-3,6-bis(4-*tert*-butyl-phenyl)-pyrrolo[3,4-c]pyrrol-1,4-dion-dibromid; 2,5-Bis[3-[N-(Methyl-imidazolium)propyl]]-3,6-bis(4-*tert*-butyl-phenyl)-pyrrolo-[3,4-c]pyrrol-1,4-dion-dibromid; 2,5-Bis[2-[N-(Methylimidazolium)-ethyl]]-3,6-bis(4-*tert*-butyl-phenyl)-pyrrolo-[3,4-c]pyrrol-1,4-dion-dibromid; 2,5-Bis[4-[N-(Methyl-imidazolium)butyl]]-3,6-bis(4-*tert*-butyl-phenyl)-pyrrolo[3,4-c]-pyrrol-1,4-dion-dichlorid; 2,5-Bis[3-[N-(Methylimidazolium)-propyl]]-3,6-bis(4-*tert*-butyl-phenyl)-pyrrolo[3,4-c]pyrrol-1,4-dion-dichlorid; 2,5-Bis[2-[N-(Methylimidazolium)ethyl]]-3,6-bis(4-*tert*-butyl-phenyl)-pyrrolo-[3,4-c]pyrrol-1,4-dion-dichlorid; 2,5-Bis[4-(N-(Methylimidazolium)-butyl]]-3,6-bis(4-chloro-phenyl)-pyrrolo[3,4-c]pyrrol-1,4-dion-dibromid; 2,5-Bis[3-[N-(Methyl-imidazolium)propyl]]-3,6-bis(4-chloro-phenyl)-pyrrolo-[3,4-c]pyrrol-1,4-dion-dibromid; 2,5-Bis[2-[N-(Methylimidazolium)-ethyl]]-3,6-bis(4-chloro-phenyl)-pyrrolo-[3,4-c]pyrrol-1,4-dion-dibromid; 2,5-Bis[4-[N-(Methyl-imidazolium)butyl]]-3,6-bis(4-chloro-phenyl)-pyrrolo[3,4-c]-pyrrol-1,4-dion-dichlorid; 2,5-Bis[3-[N-(Methylimidazolium)-propyl]]-3,6-bis(4-chloro-phenyl)-pyrrolo[3,4-c]pyrrol-1,4-dion-dichlorid; 2,5-Bis[2-[ N-(Methylimidazolium)ethyl]]-3,6-bis(4-chloro-phenyl)-pyrrolo-[3,4-c]pyrrol-1,4-dion-dichlorid; 2,5-Bis[4-[N-(Methylimidazolium)butyl]]-3,6-bis(4-methyl-phenyl)-pyrrolo[3,4-c]pyrrol-1,4-dion-dibromid; 2,5-Bis[3-[N-(Methylimidazolium)propyl]]-3,6-bis(4-methyl-phenyl)-pyrrolo-[3,4-c]pyrrol-1,4-dion-dibromid; 2,5-Bis[2-[N-(Methylimidazolium)ethyl]]-3,6-bis(4-methyl-phenyl)-pyrrolo-[3,4-c]pyrrol-1,4-dion-dibromid; 2,5-Bis[4-[N-(Methyl-imidazolium)butyl]]-3,6-bis(4-methyl-phenyl)-pyrrolo[3,4-c]-pyrrol-1,4-dion-dichlorid; 2,5-Bis[3-[N-(Methylimidazolium)-propyl]]-3,6-bis(4-methyl-phenyl)-pyrrolo[3,4-c]pyrrol-1,4-dion-dichlorid; 2,5-Bis[2-[N-(Methylimidazolium)ethyl]]-3,6-bis(4-methyl-phenyl)-pyrrolo-[3,4-c]pyrrol-1,4-dion-dichlorid; 2,5-Bis[4-[N-(Methylimidazolium)-butyl]]-3,6-bis(3-cyano-phenyl)-pyrrolo[3,4-c]pyrrol-1,4-dion-dibromid; 2,5-Bis[3-[N-(Methylimidazolium)propyl]]-3,6-bis(3-cyano-phenyl)-pyrrolo-[3,4-c]pyrrol-1,4-diondibromid; 2,5-Bis[2-[N-(Methylimidazolium)ethyl]]-3,6-bis(3-cyano-phenyl)-pyrrolo-[3,4-c]pyrrol-1,4-dion-dibromid; 2,5-Bis[4-[N-(Methylimidazolium)-butyl]]-3,6-bis(3-cyano-phenyl)-pyrrolo[3,4-c]-pyrrol-1,4-dion-dichlorid; 2,5-Bis[3-[N-(Methylimidazolium)-propyl]]-3,6-bis(3-cyano-phenyl)-pyrrolo[3,4-c]pyrrol-1,4-dion-dichlorid; 2,5-Bis[2-[N-(Methylimidazolium)-ethyl]]-3,6-bis(3-cyano-phenyl)-pyrrolo-[3,4-c]pyrrol-1,4-dion-dichlorid; 2,5-Bis[4-[N-(Methylimidazolium)butyl]]-3,6-bis(4-cyano-phenyl)-pyrrolo[3,4-c]pyrrol-1,4-dion-dibromid; 2,5-Bis[3-[N-(Methylimidazolium)-propyl]]-3,6-bis(4-cyano-phenyl)-pyrrolo-[3,4-c]pyrrol-1,4-dion-dibromid; 2,5-Bis[2-[N-(Methylimidazolium)ethyl]]-3,6-bis(4-cyano-phenyl)-pyrrolo-[3,4-c]pyrrol-1,4-dion-dibromid; 2,5-Bis[4-[N-(Methyl-imidazolium)butyl]]-3,6-bis(4-cyano-phenyl)-pyrrolo[3,4-c]-pyrrol-1,4-dion-dichlorid; 2,5-Bis[3-[N-(Methylimidazolium)-propyl]]-3,6-bis(4-cyano-phenyl)-pyrrolo[3,4-c]pyrrol-1,4-dion-dichlorid; 2,5-Bis[2-[N-(Methylimidazolium)ethyl]]-3,6-bis(4-cyano-phenyl)-pyrrolo-[3,4-c]pyrrol-1,4-dion-dichlorid; 2,5-Bis[4-[N-(Methylimidazolium)butyl]]-3,6-bis(3,4-dichloro-phenyl)-pyrrolo[3,4-c]pyrrol-1,4-dion-dibromid; 2,5-Bis[3-[N-(Methyl-imidazolium)propyl]]-3,6-bis(3,4-dichloro-phenyl)-pyrrolo-[3,4-c]pyrrol-1,4-dion-dibromid; 2,5-Bis[2-[N-(Methylimidazolium)ethyl]]-3,6-bis(3,4-dichloro-phenyl)-pyrrolo-[3,4-c]pyrrol-1,4-dion-dibromid; 2,5-Bis[4-[N-(Methyl-imidazolium)butyl]]-3,6-bis(3,4-dichloro-phenyl)-pyrrolo[3,4-c]-pyrrol-1,4-dion-dichlorid; 2,5-Bis[3-[N-(Methylimidazolium)-propyl]]-3,6-bis(3,4-dichloro-phenyl)-pyrrolo[3,4-c]pyrrol-1,4-dion-dichlorid; 2,5-Bis[2-[N-(Methylimidazolium)ethyl]]-3,6-bis(3,4-dichloro-phenyl)-pyrrolo-[3,4-c]pyrrol-1,4-dion-dichlorid; 2,5-Bis[4-[N-(Methylimidazolium)butyl]]- 3,6-bis(biphenyl)-pyrrolo[3,4-c]pyrrol-1,4-dion-dibromid; 2,5-Bis[3-[N-(Methyl-imidazolium)propyl]]-3,6-bis(biphenyl)-pyrrolo-[3,4-c]pyrrol-1,4-dion-dibromid; 2,5-Bis[2-[N-(Methylimidazolium)-ethyl]]- 3,6-bis(biphenyl)-pyrrolo-[3,4-c]pyrrol-1,4-dion-dibromid; 2,5-Bis[4-[N-(Methyl-imidazolium)butyl]]- 3,6-bis(biphenyl)-pyrrolo[3,4-c]-pyrrol-1,4-dion-dichlorid; 2,5-Bis[3-[N-(Methylimidazolium)-propyl]]-3,6-bis(biphenyl)-pyrrolo[3,4-c]pyrrol-1,4-dion-dichlorid; 2,5-Bis[2-[ N-(Methylimidazolium)-ethyl]]- 3,6-bis(biphenyl)-pyrrolo-[3,4-c]pyrrol-1,4-dion-dichlorid; 2,5-Bis(4-Trimethylammonium-butyl)-3,6-bis(3,4-dichloro-phenyl)-pyrrolo[3,4-c]pyrrol-1,4-dion-dibromid; 2,5-Bis(3-Trimethyl-ammoniumpropyl)-3,6-bis(3,4-dichloro-phenyl)-pyrrolo[3,4-c]pyrrol-1,4-dion-dibromid; 2,5-Bis(2-Trimethylammonium-ethyl)-3,6-bis(3,4-dichloro-phenyl)-pyrrolo[3,4-c]pyrrol-1,4-dion-dibromid; 2,5-Bis(4-Trimethyl-ammoniumbutyl)-3,6-bis(3,4-dichloro-phenyl)-pyrrolo[3,4-c]pyrrol-1,4-dion-dichlorid; 2,5-Bis(3-Trimethylammonium-propyl)-3,6-bis(3,4-dichloro-phenyl)-pyrrolo[3,4-c]pyrrol-1,4-dion-dichlorid; 2,5-Bis(2-Trimethyl-ammoniumethyl)-3,6-bis(3,4-dichloro-phenyl)-pyrrolo-[3,4-c]pyrrol-1,4-dion-dichlorid; 2,5-Bis[4-[4-(Dimethylamino)pyridinium]-butyl]-3,6-bis(3,4-dichlorophenyl)-pyrrolo[3,4-c]pyrrol-1,4-dion-dibromid; 2,5-Bis[3-[4-(Dimethylamino)-pyridinium]-propyl]-3,6-bis(3,4-dichloro-phenyl)-pyrrolo[3,4-c]pyrrol-1,4-dion-dibromid; 2,5-Bis[2-[4-(Dimethyl-amino)-pyridinium]ethyl]-3,6-bis(3,4-dichloro-phenyl)-pyrrolo[3,4-c]pyrrol-1,4-diondibromid; 2,5-Bis[4-[4-(Dimethylamino)-pyridinium]butyl]-3,6-bis(3,4-dichloro-phenyl)-pyrrolo[3,4-c]-pyrrol-1,4-dion-dichlorid; 2,5-Bis[3-[4(Dimethylamino)pyridinium]propyl]-3,6-bis(3,4-dichlorophenyl)-pyrrolo[3,4-c]pyrrol-1,4-dion-dichlorid; 2,5-Bis[2-[4-(Dimethylamino)-pyridinium]ethyl]-3,6-bis(3,4-dichloro-phenyl)-pyrrolo[3,4-c]-pyrrol-1,4-dion-dichlorid; 2,5-Bis(4-Trimethylammonium-butyl)-3,6-bis(4-cyanophenyl)-pyrrolo[3,4-c]pyrrol-1,4-dion-dibromid; 2,5-Bis(3-Trimethylammonium-propyl)-3,6-bis(4-cyano-phenyl)-pyrrolo[3,4-c]pyrrol-1,4-dion-dibromid; 2,5-Bis(2-Trimethylammonium-ethyl)-3,6-bis(4-cyanophenyl)-pyrrolo[3,4-c]pyrrol-1,4-dion-dibromid; 2,5-Bis(4-Trimethylammonium-butyl)-3,6-bis(4-cyano-phenyl)-pyrrolo[3,4-c]pyrrol-1,4-diondichlorid; 2,5-Bis(3-Trimethylammonium-propyl)-3,6-bis(4-cyano-phenyl)-pyrrolo[3,4-c]pyrrol-1,4-dion-dichlorid; 2,5-Bis(2-Trimethylammoniumethyl)-3,6-bis(4-cyano-phenyl)-pyrrolo-[3,4-c]pyrrol-1,4-dion-dichlorid; 2,5-Bis[4-[4-(Dimethylamino)pyridinium]butyl]-3,6-bis(4-cyano-phenyl)-pyrrolo[3,4-c]pyrrol-ol-1,4-dion-dibromid; 2,5-Bis[3-[4-(Dimethylamino)-pyridinium]propyl]-3,6-bis(4-cyano-phenyl)-pyrrolo[3,4-c]pyrrol-1,4-diondibromid; 2,5-Bis[2-[4-(Dimethylamino)pyridinium]ethyl]-3,6-bis(4-cyanophenyl)-pyrrolo[3,4-c]pyrrol-1,4-dion-dibromid; 2,5-Bis[4-[4-(Dimethylamino)pyridinium]butyl]-3,6-bis(4-cyano-phenyl)pyrrolo[3,4-c]-pyrrot-1,4-dion-dichlorid; 2,5-Bis[3-[4-(Dimethylamino)pyridinium]propyl]-3,6-bis(4-cyano-phenyl)-pyrrolo[3,4-c]pyrrol-1,4-dion-dichlorid; 2,5-Bis[2-[4-(Dimethylamino)pyridinium]ethyl]-3,6-bis(4-cyano-phenyl)-pyrrolo[3,4-c]-pyrrol-1,4-dion-dichlorid; 2,5-Bis(4-Trimethylammonium-butyl)-3,6-bis(3-cyano-phenyl)-pyrrolo[3,4-c]pyrrol-1,4-dion-dibromid; 2,5-Bis(3-Trimethylammonium-propyl)-3,6-bis(3-cyano-phenyl)-pyrrolo[3,4-c]pyrrol-1,4-diondibromid; 2,5-Bis(2-Trimethylammonium-ethyl)-3,6-bis(3-cyano-phenyl)-pyrrolo[3,4-c]pyrrol-1,4-dion-dibromid; 2,5-Bis(4-Trimethyl-ammoniumbutyl)-3,6-bis(3-cyano-phenyl)-pyrrolo[3,4-c]pyrrol-1,4-dion-dichlorid; 2,5-Bis(3-Trimethylammonium-propyl)-3,6-bis(3-cyano-phenyl)-pyrrolo[3,4-c]pyrrol-1,4-dion-dichlorid; 2,5-Bis(2-Trimethylammonium-ethyl)-3,6-bis(3-cyano-phenyl)-pyrrolo-[3,4-c]pyrrol-1,4-dion-dichlorid; 2,5-Bis[4-[4-(Dimethylamino)pyridinium]butyl]-3,6-bis(3-cyano-phenyl)-pyrrolo[3,4-c]pyrrol-1,4-dion-dibromid; 2,5-Bis[3-[4-(Dimethylamino)-pyridinium]-propyl]-3,6-bis(3-cyano-phenyl)-pyrrolo[3,4-c]pyrrol-1,4-dion-dibromid; 2,5-Bis[2-[4-(Dimethylamino)pyridinium]ethyl]-3,6-bis(3-cyano-phenyl)-pyrrolo[3,4-c]pyrrol-1,4-dion-dibromid; 2,5-Bis[4-[4-(Dimethylamino)-pyridinium]butyl]-3,6-bis(3-cyano-phenyl)-pyrrolo[3,4-c]-pyrrol-1,4-diondichlorid; 2,5-Bis[3-[4-(Dimethylamino)pyridinium]propyl]-3,6-bis(3-cyanophenyl)-pyrrolo[3,4-c]pyrrol-1,4-dion-dichlorid; 2,5-Bis[2-[4-(Dimethylamino)pyridinium)ethyl]-3,6-bis(3-cyano-phenyl)-pyrrolo[3,4-c]-pyrrol-1,4-dion-dichlorid.

Besonders bevorzugte Verbindungen der Formel (I) sind 2,5-Bis(4-Trimethylammonium-butyl)-3,6-diphenyl-pyrrolo[3,4-c]pyrrol-1,4-diondibromid, 2,5-Bis(4-Trimethylammonium-butyl)-3,6-diphenyl-pyrrolo[3,4-c]pyrrol-1,4-dion-dichlorid, 2,5-Bis(4-Trimethylammonium-butyl)-3,6-bis[4-(dimethylamlno)phenyl]-pyrrolo[3,4-c]pyrrol-1,4-dion-dibromid, 2,5-Bis(4-Trimethylammonium-butyl )-3,6-bis[4-(dimethylamino)phenyl]-pyrrolo[3,4-c]pyrrol-1,4-dion-dichlorid, 2,5-Bis[4-[4-(Dimethylamino)pyridinium]butyl]-3,6-diphenyl-pyrrolo[3,4-c]pyrrol-1,4-dion-dibromid; 2,5-Bis[4-[4-(Dimethylamino)pyridinium]butyl]-3,6-diphenyl-pyrrolo[3,4-c]pyrrol-1,4-dion-dichlorid; 2,5-Bis[4-[N-(Methylimidazolium)butyl]]-3,6-diphenyl-pyrrolo[3,4-c]pyrrol-1,4-dion-dibromid und 2,5-Bis[4-[N-(Methylimidazolium)butyl]]-3,6-diphenyl-pyrrolo[3,4-c]pyrrol-1,4-dion-dichlorid.

Die erfindungsgemäßen Farbstoffderivate der Formel (I) sind auf einfache Weise durch Einführung einer kationischen Gruppe in die Diketopyrrolopyrrole der Formel (II) herstellbar, wobei die Reste Q₁ und Q₂ die gleiche Bedeutung wie in Formel (I) haben. 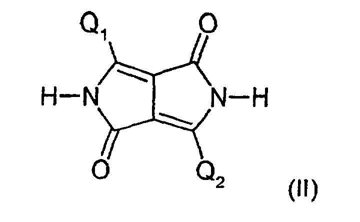 Gegenstand der vorliegenden Erfindung ist daher ebenfalls ein Verfahren zur Herstellung der kationischen Pyrrolo-pyrrol-Derivate der Formel (I) gemäss Schema 1, bei dem zunächst die NH-Gruppe in der Verbindung der Formel (II) mit einem Alkyldihalogenid umgesetzt wird und anschliessend das erhaltene Halogenderivat mit einem geeigneten tertiären Amin oder einer geeigneten tertiären phosphororganischen Verbindung ("tertiäres Phosphin") umgesetzt wird.

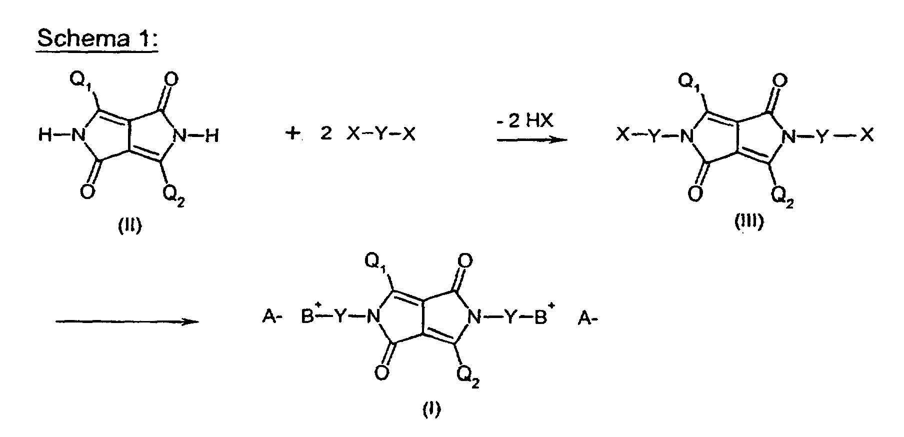

Die Herstellung der Verbindungen der Formel (I) erfolgt ausgehend von käuflichen oder leicht herstellbaren Verbindungen der Formel (II) durch Umsetzung der Verbindung der Formel (II) mit dem entsprechenden Alkyldihalogenid in Anwesenheit einer Base, wie zum Beispiel Natriumhydrid, in einem aprotischen polaren Lösungsmittel wie zum Beispiel Dimethylformamid, Dimethylacetamid, N-Methylpyrrolidon oder Dimethylsulfoxid, wobei die NH-Gruppe halogeniert wird. Anschliessend wird die erhaltene Verbindung der Formel (III) mit einem tertiären Amin oder einem tertiären Phospin zum kationischen Pyrrolo-pyrrol-Derivat der Formel (I) umgesetzt. Die Umsetzung der Verbindung der Formel (III) mit dem tertiären Amin oder dem tertiären Phospin erfolgt vorzugsweise in einem Lösungsmittel, wie zum Beispiel Ethanol, Toluol, Dioxan, Dimethylformamid, Dimethylacetamid, N-Methylpyrrolidon, Acetonitril oder Dimethylsulfoxid. Das Herstellungsverfahren gemäss Schema 1 kann hierbei sowohl einstufig (das heißt ohne Isolierung der Verbindung der Formel (III)) als auch zweistufig (das heißt mit Isolierung der Verbindung der Formel (III)) erfolgen.

Ebenfalls ist es möglich, die kationischen Pyrrolo-pyrrol-Derivate der Formel (I) durch ein einstufiges Verfahren gemäss Schema 2 herzustellen, bei dem in den Verbindungen der Formel (II) die NH-Gruppe mit einer vorgebildeten kationischen Kette, wie zum Beispiel Brombutan-4-trimethylammoniumbromid, umgesetzt wird. Das Verfahren gemäss Schema 1 ist jedoch bevorzugt.

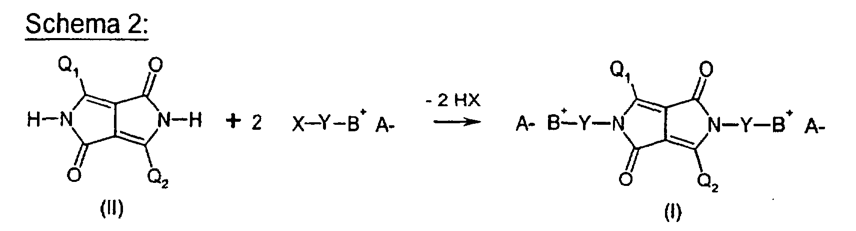

Die in Schema 1 und Schema 2 verwendeten Restgruppen z, Q₁, Q₂, Y, A⁻ und B⁺ haben die gleiche Bedeutung wie in Formel (I), während A für eine dem Anion A⁻ entsprechende ungeladene Gruppe und B für ein entsprechendes tertiäres Amin oder Phospin steht und X einem Halogenatom, vorzugsweise einem Chloratom oder Bromatom, entspricht.

Sofern das Alkyldihalogenid (Verfahren gemäss Schema 1) beziehungsweise die vorgebildete kationische Kette (Verfahren gemäss Schema 2) in einem molaren Unterschuss eingesetzt wird, können durch diese Verfahren auch die Verbindungen der Formel (I) mit R1 oder R2 gleich Wasserstoff hergestellt werden.
Als Verbindungen der Formel (II) können beispielsweise die folgenden Verbindungen eingesetzt werden:
2,5-dihydro-3,6-diphenyl-pyrrolo[3,4-c]pyrrol-1,4-dion;
3,6-bis[1,1'-(biphenyl)-4-yl]-2,5-dihydro-pyrrolo[3,4-c]pyrrol-1,4-dion;
3,6-bis(4-chlorophenyl)2,5-dihydro-pyrrolo[3,4-c]pyrrol-1,4-dion;
3,6-bis(3-chlorophenyl)-2,5-dihydro-pyrrolo[3,4-c]pyrrol-1,4-dion;
3,6-bis(4-methylphenyl)-2,5-dihydro-pyrrolo[3,4-c]pyrrol-1,4-dion;
3,6-bis(3-methylphenyl)-2,5-dihydro-pyrrolo[3,4-c]pyrrol-1,4-dion;
3,6-bis(4-tert-butylphenyl)-2,5-dihydro-pyrrolo[3,4-c]pyrrol-1,4-dion;
3,6-bis[4-(dimethylamino)phenyl]-2,5-dihydro-pyrrolo[3,4-c]pyrrol-1,4-dion;
3,6-bis(4-cyanophenyl)-2,5-dihydro-pyrrolo[3,4-c]pyrrol-1,4-dion;
3,6-bis(3-cyanophenyl)-2,5-dihydro-pyrrolo[3,4-c]pyrrol-1,4-dion;
3,6-bis(3,4-dichlorophenyl)-2,5-dihydro-pyrrolo[3,4-c]pyrrol-1,4-dion;
3,6-bis(4-methoxyphenyl)-2,5-dihydro-pyrrolo[3,4-c]pyrrol-1,4-dion und
3,6-bis(3-methoxyphenyl)-2,5-dihydro-pyrrolo[3,4-c]pyrrol-1,4-dion.

Die neuen kationischen Pyrrolo-pyrrol-Derivate der Formel (I) besitzen eine ausgezeichnete Wasserlöslichkeit und ermöglichen eine gleichmässige Färbung von Keratinfasern mit einer guten Stabilität gegen Licht, Schweiß und Shampoonieren. Die erfindungsgemässen Derivate der Formel (I) ermöglichen eine intensive, brilliante Färbung von Keratinfasem, insbesondere von menschlichen Haaren, aber auch von Wolle oder Pelzen, unter schonenden und hautverträglichen Bedingungen, wobei die erhaltene Färbung bei besonderer Anregung, beispielsweise durch UV-Licht oder Sonnenlicht, fluoreszieren kann.

Ein weiterer Gegenstand der vorliegenden Erfindung ist daher ein Mittel zum Färben von Keratinfasern, insbesondere menschlichen Haaren, welches dadurch gekennzeichnet ist; daß es mindestens ein kationisches Pyrrolo-pyrrol-Derivat der allgemeinen Formel (I) enthält.

Die kationischen Pyrrolo-pyrrol-Derivate der Formel (I) sind in dem erfindungsgemäßen Färbemittel vorzugsweise in einer Menge von 0,01 bis 10 Gewichtsprozent, insbesondere 0,1 bis 8 Gewichtsprozent, enthalten.

Das erfindungsgemäße Färbemittel kann neben den Farbstoffen der Formel (I) zusätzlich noch weitere bekannte direktfärbende Farbstoffe aus der Gruppe bestehend aus Pflanzenfarbstoffen, Nitrofarbstoffen, Azofarbstoffen, Anthrachinonfarbstoffen und Triphenylmethanfarbstoffen, alleine oder im Gemisch miteinander, enthalten, wie zum Beispiel Henna, Indigo, Kamillenblüten, Curcuma-Wurzeln, Rhabarber, Faulbaumrinde, Olivenblätter, kanadische Blutwurzel, Gelbwurzel, Gelbholz, Rotholz, Rotsandelholz, Blauholz, Krappwurzel, Schwarzer Holunder oder Schwarze Apfelbeere, 1,4-Bis[(2-hydroxyethyl)amino]-2-nitrobenzol; 1-Amino-3-methyl-4-[(2-hydroxyethyl)amino]-6-nitrobenzol (HC Violet No. 1); 1-(2-Hydroxyethyl)-amino-2-nitro-4-[di(2-hydroxyethyl)amino]-benzol (HC Blue No. 2); 4-[Ethyl-(2-hydroxyethyl)amino]-1-[(2-hydroxyethyl)-amino]-2-nitrobenzol-hydrochlorid (HC Blue No. 12); 4-[Di(2-hydroxyethyl)-amino]-1-[(2-methoxyethyl)amino]-2-nitrobenzol (HC Blue No. 11); 1-[(2,3-Dihydroxypropyl)amino]-4-[methyl-(2-hydroxyethyl)amino]-2-nitrobenzol (HC Blue No. 10); 1-[(2,3-Dihydroxypropyl)amino]-4-[ethyl-(2-hydroxy-ethyl)amino]-2-nitrobenzol-hydrochlorid (HC Blue No. 9); 1-(3-Hydroxypropylamino)-4-[di(2-hydroxyethyl)amino]-2-nitrobenzol (HC Violet No. 2); 1-Methylamino-4-[methyl-(2,3-dihydroxypropyl)amino]-2-nitrobenzol (HC Blue No. 6); 2-((4-Amino-2-nitrophenyl)amino)-5-dimethylamino-benzoesäure (HC Blue No. 13); 1-(2-Aminoethylamino)-4-[di(2-hydroxyethyl)amino]-2-nitrobenzol; 4-(Di(2-hydroxyethyl)amino)-2-nitro-1-phenylamino-benzol; 4-Amino-2-nitro-diphenylamin (HC Red No. 1); 1-Amino-4-[(2-hydroxyethyl)amino]-2-nitrobenzol (HC Red No. 7); 2-Amino-4,6-dinitro-phenol; 4-Amino-1-[(2-hydroxyethyl)amino]-2-nitrobenzol (HC Red No. 3); 1-Amino-5-chlor-4-[(2,3-dihydroxypropyl)-amino]-2-nitrobenzol (HC Red No. 10); 5-Chlor-1,4-[di(2,3-dihydroxy-propyl)amino] - 2-nitrobenzol (HC Red No. 11); 1-Amino-4-[di(2-hydroxy-ethyl)amino]-2-nitrobenzol-hydrochlorid (HC Red No. 13); 7-Amino-3,4-dihydro-6-nitro-2H-1,4-benzoxazin (HC Red No. 14); 1-Amino-5-chlor-4-[(2-hydroxyethyl)-amino]-2-nitrobenzol; 1-Amino-4-((2,3-dihydroxypropyl)amino)-5-methyl-2-nitrobenzol; 4-Amino-2-nitro-1-((prop-2-en-1-yl)amino)-benzol; 4-Amino-3-nitrophenol; 4-[(2-Hydroxyethyl)-amino]-3-nitrophenol; 1-[(2-Aminoethyl)-amino]-4-(2-hydroxyethoxy)-2-nitrobenzol (HC Orange No. 2); 4-(2,3-Dihydroxypropoxy)-1-[(2-hydroxy-ethyl)amino]-2-nitrobenzol (HC Orange No. 3); 2-[(2-Hydroxyethyl)amino]-4,6-dinitro-phenol; 4-Ethylamino-3-nitrobenzoesäure; 2-[(4-Amino-2-nitrophenyl)amino]-benzoesäure; 2-Chlor-6-ethylamino-4-nitrophenol; 2-Amino-6-chlor-4-nitrophenol; 4-[(3-Hydroxypropyl)amino]-3-nitrophenol; 2,5-Diamino-6-nitropyridin; 6-Amino-3-((2-hydroxyethyl)amino)-2-nitropyridin; 3-Amino-6-((2-hydroxyethyl)amino)-2-nitropyridin; 3-Amino-6-(ethylamino)-2-nitropyridin; 3-((2-Hydroxyethyl)-amino)-6-(methylamino)-2-nitropyridin; 3-Amino-6-(methylamino)-2-nitropyridin; 6-(Ethylamino)-3-((2-hydroxyethyl)amino)-2-nitropyridin; 1,2,3,4-Tetra-hydro-6-nitrochinoxalin; 4-[(2-Hydroxyethyl)-amino]-3-nitro-1-methylbenzol; 1-[(2-Hydroxyethyl)-amino]-2-nitrobenzol (HC Yellow No. 2); 1-(2-Hydroxyethoxy)-2-[(2-hydroxyethyl)amino]-5-nitrobenzol (HC Yellow No. 4); 1-Amino-2-[(2-hydroxyethyl)amino]-5-nitrobenzol (HC Yellow No. 5); 4-[(2,3-Dihydroxy-propyl)amino]-3-nitro-1-trifluormethyl-benzol (HC Yellow No. 6); 2-[(2-Hydroxyethyl)amino]-1-methoxy-5-nitrobenzol; 2-Amino-3-nitro-phenol; 2,3-(Dihydroxypropoxy)-3-methylamino-4-nitrobenzol; 1-Amino-4-((2-aminoethyl)amino)-5-methyl-2-nitrobenzol; 3-[(2-Aminoethyl)amino]-1-methoxy-4-nitrobenzol-hydrochlorid (HC Yellow No.9); 1-[(2-Ureidoethyl)-amino]-4-nitrobenzol; 1-Chlor-2,4-bis[(2-hydroxyethyl)-amino]-5-nitro-benzol (HC Yellow No. 10); 2-[(2-Hydroxyethyl)amino]-5-nitrophenol (HC Yellow No. 11); 1-Chlor-4-[(2-hydroxyethyl)amino]-3-nitrobenzol (HC Yellow No. 12); 4-[(2-Hydroxyethyl)amino]-3-nitro-1-trifluormethyl-benzol (HC Yellow No. 13); 4-[(2-Hydroxyethyl)-amino]-3-nitro-benzonitril (HC Yellow No. 14); 4-[(2-Hydroxyethyl)amino]-3-nitro-benzamid (HC Yellow No. 15); 1,4-Di[(2,3-dihydroxypropyl)amino]-9,10-anthrachinon; 1-[(2-Hydroxyethyl)amino]-4-methylamino-9,10-anthrachinon (C161505, Disperse Blue No. 3); 2-[(2-Aminoethyl)-amino]-9,10-anthrachinon (HC Orange No. 5); 1-Hydroxy-4-[(4-methyl-2-sulfophenyl)amino]-9,10-anthrachinon; 1-[(3-Aminopropyl)amino]-4-methylamino-9,10-anthrachinon (HC Blue No. 8); 1-[(3-Aminopropyl)-amino]-9,10-anthrachinon (HC Red No. 8); 1,4-Diamino-2-methoxy-9,10-anthrachinon (CI62015, Disperse Red No. 11); 1,4-Dihydroxy-5,8-bis[(2-hydroxyethyl)amino]-9,10-anthrachinon (Cl62500, Disperse Blue No. 7); 1,4-Diamino-9,10-anthrachinon (Cl61100, Disperse Violet No. 1); 1-Amino-4-(methylamino)-9,10-anthrachinon (Cl61105, Disperse Violet No. 4); 9-(Dimethylamino)-benzo[a]phenoxazin-7-ium-chlorid (Cl 51175; Basic Blue No. 6); Di[4-(diethylamino)phenyl][4-(ethylamino)naphthyl]carbeniumchlorid (CI 42595; Basic Blue No. 7); Di-(4-(dimethylamino)phenyl)-(4-(methyl-phenylamino)naphthalin-1-yl)carbenium-chlorid (Cl42563; Basic Blue No. 8); 3,7-Di(dimethylamino)phenothiazin-5-ium-chlorid (Cl 52015; Basic Blue No. 9); Di[4-(dimethylamino)phenyl]-[4-(phenylamino)naphthyl]-carbenium-chlorid (CI 44045; Basic Blue No. 26); 2-[(4-(Ethyl(2-hydroxyethyl)amino)phenyl)azo]-6-methoxy-3-methyl-benzothiazolium-methylsulfat (CI 11154; Basic Blue No. 41); 8-Amino-2-brom-5-hydroxy-4-imino-6-[(3-(trimethylammonio)phenyl)-amino]-1(4H)-naphthalinon-chlorid (Cl 56059; Basic Blue No. 99); Bis[4-(dimethylamino)phenyl][4-(methylamino)phenyl]-carbenium-chlorid (Cl 42535; Basic Violet No. 1); Tri(4-amino-3-methylphenyl)carbenium-chlorid (Cl42520; Basic Violet No. 2); Tris[4-(dimethylamino)phenyl)carbenium-Chlorid (Cl 42555; Basic Violet No. 3); 2-[3,6-(Diethylamino)dibenzo-pyranium-9-yl]-benzoesäure-chlorid (CI 45170; Basic Violet No. 10); Di(4-aminophenyl)(4-amino-3-methylphenyl)carbenium-Chlorid (Cl 42510; Basic Violet No. 14); 1,3-Bis[(2,4-diamino-5-methylphenyl)azo]-3-methyl-benzol (Cl 21010; Basic Brown No. 4); 1-[(4-Aminophenyl)azo]-7-(trimethylammonio)-2-naphthol-Chlorid (CI 12250; Basic Brown No. 16); 1-[(4-Amino-2-nitrophenyl)azo]-7-(trimethylammonio)-2-naphthol-chlorid (Basic Brown No. 17); 1-[(4-Amino-3-nitrophenyl)azo]-7-(trimethyl-ammonio)-2-naphthol-Chlorid (Cl 12251; Basic Brown No. 17); 3,7-Diamino-2,8-dimethyl-5-phenylphenaziniumchlorid (Cl 50240; Basic Red No. 2); 1,4-Dimethyl-5-[(4-(dimethylamino)-phenyl)azo]-1,2,4-triazolium-chlorid (Cl 11055; Basic Red No. 22); 2-Hydroxy-1-[(2-methoxyphenyl)azo]-7-(trimethylammonio)-naphthalinchlorid (Cl 12245; Basic Red No. 76); 2-[2-((2,4-Dimethoxyphenyl)amino)-ethenyl]-1,3,3-trimethyl-3H-indol-1-ium-chlorid (Cl 48055; Basic Yellow No. 11); 3-Methyl-1-phenyl-4-[(3-(trimethylammonio)phenyl)azo]-pyrazol-5-on-Chlorid (Cl 12719; Basic Yellow No. 57); Bis[4-(diethylamino)-phenyl]-phenylcarbenium-hydrogensulfat(1:1) (Cl 42040; Basic Green No. 1); Di(4-(dimethylamino)phenyl)-phenylmethanol (Cl42000; Basic Green No. 4); Di(4-(dimethylamino)phenyl)iminomethan-hydrochlorid (Cl41000; Basic Yellow No. 2); 4-[(4-Aminophenyl)azo]-1-[di(2-hydroxyethyl)amino]-3-methylbenzol (HC Yellow No. 7) oder 2,6-Diamino-3-((pyridin-3-yl)azo)-pyridin.

Die vorstehend genannten direktziehenden Farbstoffe können in einer Gesamtmenge von etwa 0,01 bis 4 Gewichtsprozent enthalten sein, wobei der Gesamtgehalt an Farbstoffen in dem erfindungsgemässen Färbemittel vorzugsweise etwa 0,01 bis 10 Gewichtsprozent, insbesondere 0,1 bis 5 Gewichtsprozent, beträgt.

Das erfindungsgemäße Färbemittel kann weiterhin alle für derartige Zubereitungen bekannten und üblichen Zusatzstoffe, beispielsweise Parfümöle, Komplexbildner, Wachse, Konservierungsstoffe, Verdicker, Alginate, Guar Gum, haarpflegende Substanzen, wie zum Beispiel kationische Polymere oder Lanolinderivate, oder anionische, nichtionische, amphotere oder kationische oberflächenaktive Substanzen enthalten. Vorzugsweise werden amphotere oder nicht-ionische oberflächenaktive Substanzen, beispielsweise Betaintenside, Propionate und Glycinate, wie zum Beispiel Cocoamphoglycinate oder Cocoamphodiglycinate, ethoxylierte Tenside mit 1 bis 1000 Ethylenoxid-Einheiten, vorzugsweise mit 1 bis 300 Ethylenoxid-Einheiten, wie zum Beispiel Glyceridalkoxylate, beispielsweise mit 25 Ethylenoxid-Einheiten ethoxyliertes Rizinusöl, Polyglykolamide, ethoxylierte Alkohole und ethoxylierte Fettalkohole (Fettalkoholalkoxylate) und ethoxylierte Fettsäurezuckerester, insbesondere ethoxylierte Sorbitanfettsäureester, eingesetzt. Die vorgenannten Bestandteile werden in den für solche Zwecke üblichen Mengen verwendet, zum Beispiel die oberflächenaktiven Substanzen in einer Konzentration von 0,1 bis 30 Gewichtsprozent, und die Pflegestoffe in einer Menge von 0,1 bis 5 Gewichtsprozent.

Das erfindungsgemäße Färbemittel kann neben Wasser auch organische Lösungsmittel, wie zum Beispiel aliphatische oder aromatische Alkohole, wie zum Beispiel Ethanol, Isopropanol, 1,2-Propandiol, 1-Methoxypropan-2-ol, 1-Ethoxy-propan-2-ol, Diethylenglykolmonomethylether oder Diethylenglykolmono-ethylether, Dipropylenglykolmonomethylether, Dipropylenglykolmonoethyl-ether, Benzylalkohol, Benzyloxyethanol, Phenylethylalkohol, Phenoxyethanol, Zimtalkohol und Glykolether, insbesondere Ethanol, enthalten, wobei der Wassergehalt in der Regel etwa 25 bis 95 Gewichtsprozent, vorzugsweise 30 bis 85 Gewichtsprozent, beträgt, während der Gehalt an dem organischen Lösungsmittel oder Lösungsmittelgemisch bei etwa 5 bis 30 Gewichtsprozent liegt.

Das erfindungsgemäße Färbemittel kann, insbesondere wenn es ein Haarfärbemittel ist, in Form einer wäßrigen oder wäßrig-alkoholischen Lösung, einer Creme, eines Gels, einer Emulsion oder eines Aerosolschaumes vorliegen, wobei das Haarfärbemittel sowohl in Form eines Einkomponentenpräparates als auch in Form eines Mehrkomponentenpräparates, beispielsweise in Form eines Zweikomponentenpräparates, bei dem das Farbstoffderivat der Formel (I) getrennt von den übrigen Bestandteilen abgepackt wird und die Herstellung des gebrauchsfertigen Haarfärbemittels erst unmittelbar vor der Anwendung durch Vermischen der beiden Komponenten erfolgt, konfektioniert sein kann.

Das erfindungsgemässe Färbemittel weist einen pH-Wert von etwa 2 bis 10, vorzugsweise etwa 5 bis 10, und insbesondere einem neutralen bis basischen pH Wert von etwa 7 bis 10, auf. Zur Einstellung des erfindungsgemässen pH-Wertes sind sowohl organische als auch anorganische Säuren oder Basen geeignet.
Als geeignete Säuren sind insbesondere die folgenden Säuren zu nennen: α-Hydroxycarbonsäuren, wie zum Beispiel Glykolsäure, Milchsäure, Weinsäure, Zitronensäure oder Äpfelsäure; Ascorbinsäure; Gluconsäurelacton; Essigsäure; Salzsäure oder Phosphorsäure, sowie Mischungen dieser Säuren.
Als geeignete Basen sind insbesonders Natriumcarbonat, Natriumhydrogencarbonat, Kaliumcarbonat, Kaliumhydrogencarbonat,
Natriumphosphat, Borax (Na₂B₄O₇ x 10H₂O), Dinatriumhydrogenphosphat, Alkanolamine, beispielsweise Monoethanolamin oder Triethanolamin, Ammoniak, Aminomethylpropanol und Natriumhydroxid zu nennen.

Die Anwendung des erfindungsgemässen Färbemittels erfolgt in der Regel indem man eine für die Haarfärbung ausreichende Menge, je nach Haarlänge etwa 30 bis 120 Gramm, des Haarfärbemittels auf das Haar aufträgt, das Haarfärbemittel bei etwa 15 bis 45 Grad Celsius etwa 1 bis 60 Minuten, vorzugsweise 5 bis 30 Minuten, einwirken läßt, das Haar anschliessend gründlich mit Wasser ausspült, gegebenenfalls mit einem Shampoo wäscht und abschliessend trocknet.

Das vorstehend beschriebene Färbemittel kann weiterhin für kosmetische Mittel übliche natürliche oder synthetische Polymere beziehungsweise modifizierte Polymere natürlichen Ursprungs enthalten, wodurch gleichzeitig mit der Färbung eine Festigung der Haare erreicht wird. Solche Mittel werden im allgemeinen als Tönungsfestiger oder Farbfestiger bezeichnet.

Von den für diesen Zweck in der Kosmetik bekannten synthetischen Polymeren seien beispielsweise Polyvinylpyrrolidon, Polyvinylacetat, Polyvinylalkohol oder Polyacrylverbindungen wie Polyacrylsäure oder Polymethacrylsäure, basische Polymerisate von Estern der Polyacrylsäure, Polymethylacrylsäure und Aminoalkoholen beispielsweise deren Salze oder Quaternisierungsprodukte, Polyacrylnitril, Polyvinylacetate sowie Copolymerisate aus derartigen Verbindungen, wie zum Beispiel Polyvinylpyrrolidon-Vinylacetat, erwähnt; während als natürliche Polymere oder modifizierte natürliche Polymere beispielsweise Chitosan (entacetyliertes Chitin) oder Chitosanderivate, eingesetzt werden können.

Die vorgenannten Polymere können in dem erfindungsgemäßen Mittel in der für solche Mittel üblichen Mengen, insbesondere in einer Menge von etwa 1 bis 5 Gewichtsprozent, enthalten. Der pH-Wert des erfindungsgemäßen Tönungsfestigers oder Farbfestigers beträgt vorzugsweise etwa 6 bis 9.

Die Anwendung des Haarfärbemittels mit zusätzlicher Festigung erfolgt in bekannter und üblicher Weise durch Befeuchten des Haares mit dem Festiger, Festlegen (Einlegen) des Haares zur Frisur und anschließende Trocknung.

Das erfindungsgemäße Färbemittel ermöglicht eine hervorragende, gleichmäßige, intensive und äußerst dauerhafte Färbung von Keratinfasern (beispielsweise menschlichen Haaren, Wolle oder Pelzen) ohne nennenswerte Anfärbung der Haut beziehungsweise Kopfhaut, die fünf oder mehr Haarwäschen ohne ein merkliches Verblassen der Haarfarbe überdauert.

Die nachfolgenden Beispiele sollen den Gegenstand der Erfindung näher erläutern, ohne diesen hierauf zu beschränken.

### Beispiele

### Beispiel 1: Darstellung von 2,5-Bis(4-Trimethylammonium-butyl)-3,6-diphenyl-pyrrolo[3,4-c]pyrrol-1,4-dion-dibromid

### Stufe 1: Darstellung von 2,5-Bis(4-Bromobutyl)-3,6-diphenyl-pyrrolo-[3,4-c]pyrrol-1,4-dion

5 g 2,5-Dihydro-3,6-diphenyl-pyrrolo[3,4-c]pyrrol-1,4-dion (17,3 mmol) (Formel (II) mit Q1 =Q2= Phenyl), werden in 55 ml N-Methylpyrrolidon suspendiert und unter Rühren auf 0°C abgekühlt. Bei dieser Temperatur werden 1,73 g (43,2 mmol) Natriumhydrid (60%ige Suspension in Oel) hinzugefügt und anschliessend die Mischung zunächst langsam dann kräftig gerührt. Sodann wird die Reaktionsmischung 1,5 Stunden lang bei Raumtemperatur gerührt und erneut auf 0°C abgekühlt. Die Reaktionsmischung wird mit einer Spatelspitze Kaliumjodid versetzt und sodann tropfenweise mit 29,88 g (138 mmol) Dibrombutan in 80 ml N-Methylpyrrolidon versetzt. Die Reaktionsmischung wird anschliessend über Nacht bei Raumtemperatur gerührt und sodann auf Eis gegossen.
Das ausfallende orangefarbene Produkt wird filtriert, mit Hexan und mit Wasser gewaschen und anschliessend getrocknet.
Ausbeute: 7,48 g (77% der Theorie), rotes Pulver
¹H-NMR (DMSO/300 MHz): 7,82 (d, J=7Hz, 4H aromat.), 7,58 (m, 6H aromat.), 3,73 (t, J=7,1 Hz, 4H, CH₂-N), 3,40 (t, J=6,5 Hz, 4H, CH₂-Br), 1,65 (m, 4H, CH₂), 1,54 (m, 4H, CH₂).

### Stufe 2: Darstellung von 2,5-Bis(4-Trimethylammonium-butyl)-3,6-diphenyl-pyrrolo[3,4-c]pyrrol-1,4-dion-dibromid

2 g (3,6 mmol) 2,5-Bis(4-Bromobutyl)-3,6-diphenyl-pyrrolo[3,4-c]pyrrol-1,4-dion aus Stufe 1 werden in 55 ml Toluol/Ethanol (10:1) gelöst und mit 8,5 ml (36 mmol) einer 33%igen ethanolischen Trimethylamin-Lösung versetzt. Die Reaktionsmischung wird etwa 5 Stunden bei 65°C erhitzt und sodann abgekühlt. Das ausfallende Produkt wird filtriert, mit Toluol und Essigsäure-Ethylester gewaschen und abschliessend getrocknet
Ausbeute: 2,17g (89% der Theorie), rotes Pulver
¹H-NMR (DMSO/300 MHz): 7,83 (m, 4H aromat.), 7,59 (m, 6H aromat.), 3,73 (m, 4H, CH₂-N), 3,21 (m, 4H, CH₂-N⁺), 3,0 (s, 18H, CH₃), 1,58 (m, 4H, CH₂), 1,46 (m, 4H, CH₂).
MS (ESI):258 (M²⁺/2).

Das so erhaltene Rohprodukt kann ohne weitere Reinigung direkt in dem erfindungsgemäßen Haarfärbemittel eingesetzt werden.

### Beispiel 2: Darstellung von 2,5-Bis(4-Trimethylammonium-butyl)-3,6-bis[4-(dimethylamino)phenyl]-pyrrolo[3,4-c]pyrrol-1,4-diondibromid

### Stufe 1: Darstellung von 2,5-Bis(4-bromobutyl)-3,6-bis[4-(dimethylamino)-phenyl]-pyrrolo[3,4-c]pyrrol-1,4-dion

5 g 3,6-Bis[4-(dimethylamino)phenyl]-2,5-dihydro- pyrrolo[3,4-c]pyrrol-1,4-dion (13,4 mmol) (Formel (II) mit Q1=Q2=C₆H₄-N(CH₃)₂), werden in 150 ml N-Methylpyrrolidon suspendiert und auf 0°C abgekühlt. Bei dieser Temperatur werden 1,46 g (33,5 mmol) Natriumhydrid (60%ige Suspension in Oel) hinzugegeben und die Mischung anschliessend zuerst langsam dann kräftig gerührt. Nach anderthalbstündigem Rühren bei Raumtemperatur wird die Reaktionsmischung erneut auf 0°C abgekühlt und mit einer Spatelsptize Kaliumjodid versetzt. Sodann werden unter Rühren 23,1 g (106,8 mmol) Dibrombutan tropfenweise hinzugegeben und die Reaktionsmischung über Nacht bei Raumtemperatur weitergerührt. Anschliessend wird die Reaktionsmischung auf Wasser gegossen und das erhaltene Gemisch 2 Stunden lang auf 80°C erwärmt. Das ausfallende violette Produkt wird abfiltriert, mit Hexan und Ethanol gewaschen und dann getrocknet.
Ausbeute: 5,9 g (69 % der Theorie), violettes Pulver
¹H-NMR (DMSO/300 MHz): 7,83 (d, J=9,0 Hz, 4H aromat.), 6,82 (d, J=9,0 Hz, 4H aromat.), 3,81 (m, 4H, CH₂-N), 3,47 (m, 4H, CH₂-Br), 3,03 (s, 12H, CH₃-N), 1,72 (m, 4H, CH₂), 1,65 (m, 4H, CH₂).

### Stufe 2: Darstellung von 2,5-Bis(4-Trimethylammonium-butyl)-3,6-bis[4-(dimethylamino)phenyl]-pyrrolo[3,4-c]pyrrol-1,4-dion-dibromid

4 g (6,2 mm 5-Bis(4-bromobutyl)-3,6-bis[4-(dimethylamino)-phenyl]-pyrrolo[3,4-c]pyrrol-1,4-dion aus Stufe 1 werden in 100 ml Toluol gelöst und mit 14,8 ml (62 mmol) einer 33%igen ethanolischen Trimethylamin-Lösung versetzt. Die Reaktionsmischung wird 5,5 Stunden lang bei 65°C gerührt und sodann abgekühlt. Das ausfallende Produkt wird abfiltriert, mit Toluol, Ethanol und Aceton gewaschen und abschliessend getrocknet.
Ausbeute: 4,5 g (95 % der Theorie), violettes Pulver
¹H-NMR (DMSO/300 MHz): 7,82 (d, J=8,9 Hz, 4H aromat.), 6,82 (d, J=8,9 Hz, 4H aromat.), 3,80 (m, 4H, CH₂-N), 3,30 (m, 4H, CH₂-N⁺), 3,04 (s, 12H, CH₃-N), 3,0 (s, 18H, CH₃- N⁺), 1,65 (m, 4H, CH₂), 1,54 (m, 4H, CH₂).
MS (ESI): 301 (M²⁺/2).

Das so erhaltene Rohprodukt kann ohne weitere Reinigung direkt in dem erfindungsgemäßen Haarfärbemittel eingesetzt werden.

### Beispiel 3: Darstellung von 2,5-Bis[4-[4-(Dimethylamino)pyridinium]-butyl]-3,6-diphenyl-pyrrolo[3,4-c]pyrrol-1,4-dion-dibromid

1 g (1,8 mmol) 2,5-Bis(4-Bromobutyl)-3,6-diphenyl-pyrrolo[3,4-c]pyrrol-1,4-dion aus Stufe 1 von Beispiel 1 werden in 30 ml Toluol gelöst und mit 2,2 g (18 mmol) 4-(Dimethylamino)-Pyridin versetzt. Die Reaktionsmischung wird 7,5 Stunden lang bei 55°C gerührt und sodann abgekühlt. Das ausfallende Produkt wird abfiltriert, mit Toluol und Ethanol gewaschen und abschliessend getrocknet.
Ausbeute: 0,76 g (53 % der Theorie), oranges Pulver.
¹H-NMR (DMSO/300 MHz): 8,21 (d, J=7,3 Hz, 4H Pyridinium), 7,79 (m, 4H aromat.), 7,59 (m, 6H aromat.), 6,98 (d, J=7,3 Hz, 4H Pyridinium), 4,09 (m, 4H, CH₂-N⁺), 3,75 (m, 4H, CH₂-N), 3,20 (s, 12H, N-CH₃), 1,65 (m, 4H, CH₂), 1,35 (m, 4H, CH₂).
MS (ESI): 321 (M²⁺/2).

Das so erhaltene Rohprodukt kann ohne weitere Reinigung direkt in dem erfindungsgemäßen Haarfärbemittel eingesetzt werden.

### Beispiel 4: Darstellung von 2,5-Bis[4-[N-(Methylimidazolium)butyl]]-3,6-diphenyl-pyrrolo[3,4-c]pyrrol-1,4-dion-dibromid

1 g (1.8 mmol) 2,5-Bis(4-Bromobutyl)-3,6-diphenyl-pyrrolo[3,4-c]pyrrol-1,4-dion aus Stufe 1 von Beispiel 1 werden in 33 ml Toluol/Ethanol (10:1) gelöst und mit 1,47 g (18 mmol) N-Methylimidazol versetzt. Die Reaktionsmischung wird 3 Tage lang bei 110°C gerührt und sodann abgekühlt. Das ausfallende Produkt wird abfiltriert, mit Toluol und Ethanol gewaschen und abschliessend getrocknet.
Ausbeute: 1,2 g (93 % der Theorie), oranges Pulver.
¹H-NMR (DMSO/300 MHz): 9-05 (s, 2H, H(2) Imidazolium), 7,82 (m, 4H aromat.), 7,69 (m, 4H, H(4) und H(5) Imidazolium), 7,62 (m, 6H aromat.), 4,10 (t, 4H, CH₂-N⁺), 3,82 (s, 6H, N-CH₃), 3,75 (t, 4H, CH₂-N), 1,68 (m, 4H, CH₂), 1,40 (m, 4H, CH₂).
MS (ESI): 281 (M²⁺/2).

Das so erhaltene Rohprodukt kann ohne weitere Reinigung direkt in dem erfindungsgemäßen Haarfärbemittel eingesetzt werden.

### Beispiele 5 bis 8: Haarfärbemittel

| **Färbelösung:** | |
|---|---|
| 2,5 mmol | Farbstoff der Formel (I) |
| 5,0 g | Ethanol |
| 2,0 g | Decylpolyglucose |
| 0,2 g | Ethylendiaminotetraessigsäure-Dinatriumsalz-Hydrat |
| ad 100,0 g | Wasser, vollentsalzt |

Die Färbelösung wird durch Zugabe von Ammoniak auf einen pH-Wert von 7 bis 10 eingestellt.

Die Haarfärbung erfolgt indem eine für die Haarfärbung ausreichende Menge des Färbemittels auf das Haar aufgetragen wird. Nach einer Einwirkungszeit von 30 Minuten bei 40 °C wird das Haar mit lauwarmem Wasser ausgespült, mit einem Shampoo gewaschen, nochmals mit Wasser ausgespült und getrocknet. Die Färbeergebnisse sind in der nachfolgenden Tabelle 1 zusammengefasst.

**Tabelle 1**

| **Beispiel Nr./Farbstoff der Formel (I)** | | **Fär- bung** | **L*a*b*- Farbmeß- werte** | **Fluores- zenz bei Anregung λ=366 nm** |
|---|---|---|---|---|
| **5** | 2,5-Bis(4-Trimethylammonium-butyl)-3,6-diphenyl-pyrrolo[3,4-c]pyrrol-1,4-dion-dibromid | gelb-orange | L= 71,08 a= 20,26 b= 89,58 | fluoreszierendes Gelb |
| **6** | 2,5-Bis(4-Trimethylammonium-butyl)-3,6-bis[4-(dimethylamino)phenyl]-pyrrolo[3,4-c]pyrrol-1,4-dion-dibromid | violett | L= 24,88 a= 32,85 b= - 10,53 | fluoreszierendes Rot |
| **7** | 2,5-Bis[4-[4-Dimethylamino)-pyridinium]butyl]-3,6-diphenyl-pyrrolo-[3,4-c]pyrrol-1,4-dion-dibromid | gelb-orange | L= 76,05 a= 15,95 b= 97,02 | fluoreszierendes Gelb |
| **8** | 2,5-Bis[4-[N-(Methylimidazolium)-butyl]]-3,6-diphenyl-pyrrolo-[3,4-c]-pyrrol-1,4-dion-dibromid | gelb-orange | L= 76,58 a= 15,80 b= 95,34 | fluoreszierendes Gelb |

### Beispiele 9 bis 16: Haarfärbemittel

| **Färbelösung:** | |
|---|---|
| X mmol | Farbstoff der Formel (I) (Menge gemäss Tabelle 2) |
| Y mmol | direktfärbende Farbstoffe (Art und Menge gemäss Tabelle 2) |
| 5,0 g | Ethanol |
| 2,0 g | Decylpolyglucose |
| 0,2 g | Ethylendiaminotetraessigsäure-Dinatriumsalz-Hydrat |
| ad 100,0 g | Wasser, vollentsalzt |

Die Färbelösung wird durch Zugabe von Ammoniak auf einen pH-Wert von 7 bis 10 eingestellt.

Die Haarfärbung erfolgt indem eine für die Haarfärbung ausreichende Menge des Färbemittels auf das Haar aufgetragen wird. Nach einer Einwirkungszeit von 30 Minuten bei 40 °C wird das Haar mit lauwarmem Wasser ausgespült, mit einem Shampoo gewaschen, nochmals mit Wasser ausgespült und getrocknet. Die Färbeergebnisse sind in der nachfolgenden Tabelle 2 zusammengefasst.

**Tabelle 2**

| **Beispiel Nr./Farbstoff der Formel (I)** | **Färbung** | **L*a*b*-Farbmeßwerte** |
|---|---|---|
| **9** 2,5-Bis-(4-Trimethylammonium-butyl)-3,6-diphenyl-pyrrolo[3,4-c]-pyrrol-1,4-don-dibromid(1,25 mmol) und 3-methyl-1-phenyl-4-[(3-trimethylammonium)phenyl)azo]-pyrazol-5-on-chlorid (Basic Yellow No. 57) (1,25 mmol) | Leuchtendes goldgelb | L= 69,80 a= 16,71 b= 82,55 |
| **10** 2,5-Bis-(4-Trimethylammonium-butyl)-3,6-diphenyl-pyrrolo[3,4-c]-pyrrol-1,4-don-dibromid(1,25 mmol) und 2-Hydroxy-1-[(2-methoxyphenyl)azo]-7-(trimethylammonium)-naphthalin-chlorid (Basic Red No. 76) 1,25 mmol) | Leuchtendes Orange | L= 47,75 a= 48,66 b= 48,18 |
| **11** 2,5-Bis-(4-Trimethylammonium-butyl)-3,6-diphenyl-pyrrolo[3,4-c]-pyrrol-1,4-don-dibromid(1,25 mmol) und 1-[(4-Aminophenyl)azo]-7-(trimethylammonium)-2-naphthol-chlorid (Basic Brown No. 16) (1,25 mmol) | Leuchtendes Mahgoni | L=23,10 a= 19,84 b= 10,61 |
| **12** 2,5-Bis-(4-Trimethylammonium-butyl)-3,6-diphenyl-pyrrolo[3,4-c]-pyrrol-1,4-don-dibromid(1,25 mmol) und 3,6-Bis[4-(dimethylamino)-phenyl]-pyrrolo[3,4-c]pyrrol-1,4-doin-dibromid (1,25 mmol) und 2-Hydroxy-1-[(2-methoxyphenyl)azo]-7-(trimethylammonio)-naphthalin-chlorid (Basic Red No. 76) (1,25 mmol) | Leuchtendes Kirschrot | L= 30,01 a= 35,74 b= 8,97 |
| **13** 2,5-Bis(4-Trimethylammonium-butyl)-3,6-bis[4-(dimethylamino)-phenyl]-pyrrolo-[3,4-c]pyrrol-1,4-dion-dibromid (1,25 mmol) und 8-Amin-2-brom-5-hydroxy-4-imino-6-[(3-(trimethylammonium)phenyl)-amino]-1(4H)-naphthalinon-chlorid (Basic Blue No. 99) (1,25 mmol) | Leuchtendes Blau | L= 21,81 a= 8,65 b= -12,82 |
| **14** 2,5-Bis(4-Trimethylammonium-butyl)-3,6-bis[4-(dimethylamino)-phenyl]-pyrrolo-[3,4-c]pyrrol-1,4-dion-dibromid (1,25 mmol) und 1-[(4-Aminophenyl)azo]-7-(trimethylammonium)-2-naphthol-chlorid (Basic Brown No. 16) (1,25 mmol) | Leuchtendes Braunrot | L= 20,06 a= 17,53 b= 6,47 |
| **15** 2,5-Bis(4-Trimethylammonium-butyl)-3,6-diphenyl-pyrrolo[3,4c]-pyrrol-1,4-dion-dibromid (0,41 mmol) und 3-Methyl-1-phenyl-4-[(3-(trimethylammonium)phenyl)azo]-pyrazol-5-on-chlorid (Basic Yellow No. 57) (0,41 mmol); 2-Hydroxy-1-[(2-methoxyphenyl)azo]-7-(trimethylammonium)-naphthalin-chlorid (Basic Red No. 76) (0,41 mmol); 1-[(4-Aminophenyl)azo]-7-(trimethylammonium)-2-naphthol-chlorid (Basic Brown No. 16) (0,41 mmol); 1-[(4-Amino-3-nitrophenyl)azo]-7-(trimethylammonium)-2-naphthol-chlorid (Basic Brown No. 17) (0,41 mmol); 8-Amino-2-brom-5-hydroxy-4-imino-6-[(3-(trimethylammonium)phenyl)-amino]-1 (4H)-naphthalinon-chlorid (Basic Blue No. 99) (0,41mmol) | Leuchtendes Mahagoni | L= 25,18 a= 13,52 b= 11,23 |
| **16** 2,5-Bis(4-Trimethylammonium-butyl)-3,6-diphenyl-pyrrolo[3,4-c]-pyrrol-1,4-dion-dibromid (0,41 mmol) und 3-Methyl-1-phenyl-4-[(3-(trimethylammonium)phenyl)azo]-pyrazol-5-on-chlorid (Basic Yellow No. 57) (0,41 mmol); 2-Hydroxy-1-[(2-methoxyphenyl)azo]-7-(trimethylammonium)-naphthalin-chlorid (Basic Red No. 76) (0,41 mmol); 1-[(4-Aminophenyl)azo]-7-(trimethyl-ammonium)-2-naphthol-Chlorid (Basic Brown No. 16) (0,41mmol); 1-[(4-Amino-3-nitrophenyl)azo]-7-(trimethylammonium)-2-naphthol-chlorid (Basic Brown No. 17) (0,41mmol); 8-Amino-2-brom-5-hydroxy-4-imino-6-[(3-(trimethylammonium)phenyl)-amino]-1(4H)-naphthalinon-chlorid (Basic Blue No. 99) (0,41 mmol) | Leuchtendes Braunrot | L= 23,39 a= 12,93 b= 8,98 |

Die in den vorliegenden Beispielen ermittelten L*a*b*-Farbmeßwerte wurden mit einem Farbmessgerät der Firma Minolta, Typ Chromameter II, ermittelt. Hierbei steht der L-Wert für die Helligkeit (das heißt je geringer der L-Wert ist, umso größer ist die Farbintensität), während der a-Wert ein Maß für den Rotanteil ist (das heißt je größer der a-Wert ist, umso größer ist der Rotanteil). Der b-Wert ist ein Maß für den Blauanteil der Farbe, wobei der Blauanteil umso größer ist, je negativer der b-Wert ist.

Alle in der vorliegenden Anmeldung genannten Prozentangaben stellen soweit nicht anders angegeben Gewichtsprozente dar.

## Patentansprüche

1. Kationisches Diketopyrrolopyrrol der allgemeinen Formel (I) 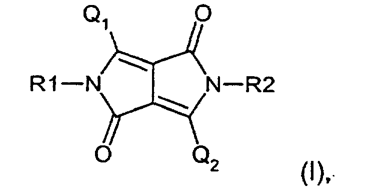 worin unabhängig voneinander Q₁ und Q₂ einen gegebenenfalls substituierten aromatischen isozyklischen oder heterozyklischen Rest mit 5 bis 14 Atomen im Ring, wobei der heterozyklische Rest mindestens ein Sauerstoff-, Stickstoff- oder Schwefelatom enthält, bedeuten und die Reste R1 und R2 unabhängig voneinander Wasserstoff oder eine Gruppe Y-B⁺ A⁻ bedeuten, unter der Bedingung, dass mindestens einer der Reste R1 und R2 nicht gleich Wasserstoff ist; B⁺ gleich einer aromatischen, aliphatischen, alizyklischen, aromatischen heterozyklischen oder nicht-aromatischen heterozyklischen, quaternären Ammoniumgruppe oder quatemären Phosphoniumgruppe ist; Y gleich einer gegebenenfalls substituierten, geradkettigen oder verzweigten C₁- bis C₆-Alkylengruppe ist und A- gleich einem Anion ist.

2. Diketopyrrolopyrrol nach Anspruch 1, **dadurch gekennzeichnet, dass** in Formel (I) B⁺ für (i) eine aromatische, heterozyklische quaternäre Ammoniumverbindung; (ii) eine nicht-aromatische heterozyklische quaternäre Ammoniumverbindung; (iii) eine quaternäre Alkylammoniumverbindung, Arylammoniumverbindung oder Arylalkylammoniumverbindung der Formel NR⁵R⁶R⁷, wobei R⁵,R⁶ und R⁷ unabhängig voneinander einen Benzylrest, einen Phenylrest, oder einen C₁- bis C₆- Alkylrest bedeuten, wobei die vorgenannten Alkylreste unsubstituiert oder mit einer oder mehreren Hydroxygruppen oder Aminogruppen substituiert sein können; oder (iv) eine quaternäre Phosphoniumverbindung stehen.

3. Diketopyrrolopyrrol nach Anspruch 1 oder 2, **dadurch gekennzeichnet, dass** in Formel (I) Y eine -CH₂-Gruppe, eine -CH₂-CH₂-Gruppe, eine -CH₂-CH₂-CH₂-Gruppe , eine -CH₂-CH₂-CH₂-CH₂-Gruppe oder einen mit einer oder mehreren Alkylgruppen, Hydroxygruppen, Aminogruppen, Acylgruppen oder quatemären Ammoniumgruppen substituierten, geradkettigen oder verzweigten C₂- bis C₆- Alkylenrest darstellt.

4. Diketopyrrolopyrrol nach einem der Ansprüche 1 bis 3, **dadurch gekennzeichnet, dass** in Formel (I) das Anion A⁻ ausgewählt ist aus lodid, Chlorid, Bromid, Sulfat, Phosphat, Hydrogenphosphat, Oxalat, Carbonat, Hydrogencarbonat, Formiat, Acetat, Zitrat, Tartrat, Malonat und Pyruvat.

5. Verfahren zur Herstellung der Diketopyrrolopyrrole der Formel (I) gemäss einem der Ansprüche 1 bis 4 bei dem eine Verbindung der Formel (II) mit dem entsprechenden Alkyldihalogenid in Anwesenheit einer Base in einem aprotischen polaren Lösungsmittel umgesetzt wird und anschliessend das erhaltene Halogenid mit einem tertiären Amin oder einem tertiären Phospin umgesetzt wird.

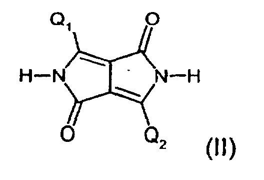

6. Verfahren zur Herstellung der Diketopyrrolopyrrole der Formel (I) gemäss einem der Ansprüche 1 bis 4 bei dem eine Verbindung der Formel (II) 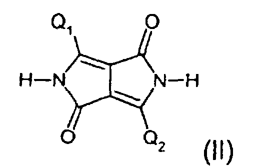 mit einer vorgebildeten kationischen Kette X-Y-B⁺ A⁻ umgesetzt wird.

7. Mittel zum Färben von Keratinfasem, **dadurch gekennzeichnet, dass** es mindestens ein Diketopyrrolopyrrol der Formel (I) gemäss einem der Ansprüche 1 bis 4 enthält.

8. Mittel nach Anspruch 7, **dadurch gekennzeichnet, dass** das Diketopyrrolopyrrol der Formel (I) ausgewählt ist aus 2,5-Bis(4-Trimethylammonium-butyl)-3,6-diphenyl-pyrrolo[3,4-c]pyrrol-1,4-dion-dibromid, 2,5-Bis(4-Trimethylammonium-butyl)-3,6-diphenyl-pyrrolo[3,4-c]pyrrol-1,4-dion-dichlorid, 2,5-Bis(4-Trimethylammonium-butyl)-3,6-bis[4-(dimethylamino)phenyl]-pyrrolo[3,4-c]pyrrol-1,4-dion-dibromid, 2,5-Bis(4-Trimethylammonium-butyl)-3,6-bis[4-(dimethylamino)phenyl]-pyrrolo[3,4-c]pyrrol-1,4-dion-dichlorid, 2,5-Bis[4-[4-(Dimethylamino)pyridinium]butyl]-3,6-diphenyl-pyrrolo[3,4-c]pyrrol-1,4-dion-dibromid; 2,5-Bis[4-[4-(Dimethylamino)pyridinium]butyl]-3,6-diphenyl-pyrrolo[3,4-c]pyrrol-1,4-dion-dichlorid; 2,5-Bis[4-[N-(Methylimidazolium)butyl]]-3,6-diphenyl-pyrrolo[3,4-c]pyrrol-1,4-dion-dibromid und 2,5-Bis[4-[N-(Methylimidazolium)butyl]]-3,6-diphenyl-pyrrolo[3,4-c]pyrrol-1,4-dion-dichlorid.

9. Mittel nach Anspruch 7 oder 8, **dadurch gekennzeichnet, dass** das Diketopyrrolopyrrol der Formel (I) in einer Menge von 0,01 bis 10 Gewichtsprozent enthalten ist.

10. Mittel nach einem der Ansprüche 7 bis 9, **dadurch gekennzeichnet, dass** es zusätzlich noch mindestens einen weiteren direktfärbende Farbstoff aus der Gruppe bestehend aus Pflanzenfarbstoffen, Nitrofarbstoffen, Azofarbstoffen, Anthrachinonfarbstoffen und Triphenylmethanfarbstoffen enthält.

11. Mittel nach einem der Ansprüche 7 bis 10, **dadurch gekennzeichnet, dass** es ein Haarfärbemittel ist.

12. Verwendung eines Diketopyrrolopyrrols der Formel (I) gemäss einem der Ansprüche 1 bis 4 als nicht-oxidativer Farbstoff in Färbemitteln für Keratinfasem.

## Claims

1. Cationic diketopyrrolopyrrole of the general formula (I) 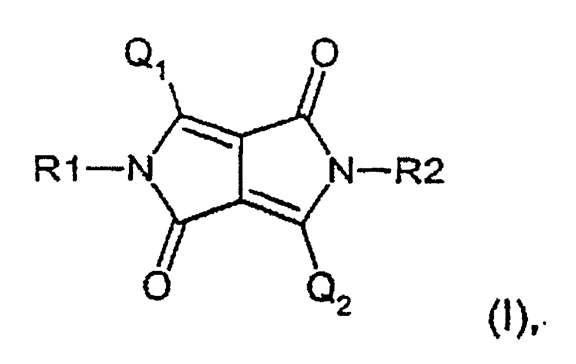 in which, independently of one another, Q₁ and Q₂ are an optionally substituted aromatic isocyclic or heterocyclic radical with 5 to 14 atoms in the ring, where the heterocyclic radical contains at least one oxygen, nitrogen or sulphur atom, and the radicals R1 and R2, independently of one another, are hydrogen or a group Y-B⁺ A⁻, under the condition that at least one of the radicals R1 and R2 is not hydrogen; B⁺ is an aromatic, aliphatic, alicyclic, aromatic heterocyclic or nonaromatic heterocyclic, quaternary ammonium group or quaternary phosphonium group; Y is an optionally substituted straight-chain or branched C₁-C₆-alkylene group, and A⁻ is an anion.

2. Diketopyrrolopyrrole according to claim 1, **characterized in that**, in formula (I), B⁺ is (i) an aromatic, heterocyclic quaternary ammonium compound; (ii) a nonaromatic heterocyclic quaternary ammonium compound; (iii) a quaternary alkyl ammonium compound, aryl ammonium compound or aryl alkyl ammonium compound of the formula NR⁵R⁶R⁷, where R⁵, R⁶ and R⁷, independently of one another, are a benzyl radical, a phenyl radical, or a C₁-C₆-alkyl radical, where the abovementioned alkyl radicals may be unsubstituted or substituted by one of more hydroxyl groups or amino groups; or (iv) a quaternary phosphonium compound.

3. Diketopyrrolopyrrole according to claim 1 or 2, **characterized in that**, in formula (I), Y is a -CH₂ group, a -CH₂-CH₂ group, a -CH₂-CH₂-CH₂ group, a -CH₂₋CH₂-CH₂-CH₂ group or a straight-chain or branched C₂-C₆-alkylene radical substituted by one or more alkyl groups, hydroxyl groups, amino groups, acyl groups or quaternary ammonium groups.

4. Diketopyrrolopyrrole according to one of claims 1 to 3, **characterized in that**, in formula (I), the anion A⁻ is chosen from iodide, chloride, bromide, sulphate, phosphate, hydrogen phosphate, oxalate, carbonate, hydrogen carbonate, formiate, acetate, citrate, tartrate, malonate and pyruvate.

5. Process for the preparation of the diketopyrrolopyrroles of the formula (I) according to one of claims 1 to 4 in which a compound of the formula (II) is reacted with the corresponding alkyl dihalide in the presence of a base in an aprotic polar solvent, and then the resulting halide is reacted with a tertiary amine or a tertiary phosphine.

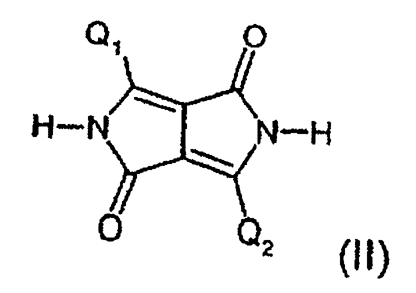

6. Process for the preparation of the diketopyrrolopyrroles of the formula (I) according to one of claims 1 to 4 in which a compound of the formula(II) 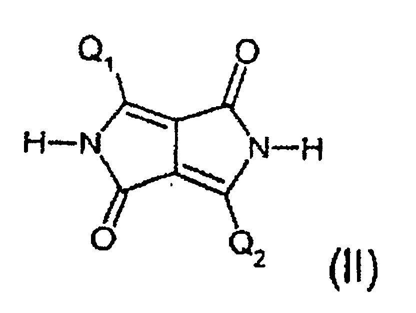 is reacted with a previously depicted cationic chain X-Y-B⁺ A⁻.

7. Agent for colouring keratin fibres, **characterized in that** it comprises at least one diketopyrrolopyrrole of the formula (I) according to one of claims 1 to 4.

8. Agent according to claim 7, **characterized in that** the diketopyrrolopyrrole of the formula (I) is chosen from 2,5-bis(4-trimethylammonium-butyl)-3,6-diphenylpyrrolo[3,4-c]pyrrole-1,4-dione dibromide, 2,5-bis(4-trimethylammonium-butyl)-3,6-diphenylpyrrolo[3,4-c]-pyrrole-1,4-dione dichloride, 2,5-bis(4-trimethylammonium-butyl)-3,6-bis[4-(dimethylamino)phenyl]-pyrrolo[3,4-c]pyrrole-1,4-dione dibromide, 2,5-bis(4-trimethylammonium-butyl)-3,6-bis[4-(dimethylamino)phenyl]pyrrolo[3,4-c]pyrrole-1,4-dione dichloride, 2,5-bis[4-[4-(dimethylamino)pyridinium]-butyl]-3,6-diphenylpyrrolo[3,4-c]pyrrole-1,4-dione dibromide; 2,5-bis[4-[4-(dimethylamino)pyridinium]-butyl]-3,6-diphenylpyrrolo[3,4-c]pyrrole-1,4-dione dichloride; 2,5-bis[4-[N-(methylimidazolium)butyl]]-3,6-diphenylpyrrolo[3,9-c]pyrrole-1,4-dione dibromide and 2,5-bis[4-[N-(methylimidazolium)butyl]]-3,6-diphenylpyrrolo[3,4-c]pyrrole-1,4-dione dichloride.

9. Agent according to claim 7 or 8, **characterized in that** the diketopyrrolopyrrole of the formula (I) is present in an amount of from 0.01 to 10% by weight.

10. Agent according to one of claims 7 to 9, **characterized in that** it additionally also comprises at least one further direct dye from the group consisting of plant dyes, nitro dyes, azo dyes, anthraquinone dyes and triphenylmethane dyes.

11. Agent according to one of claims 7 to 10, **characterized in that** it is a hair colorant.

12. Use of a diketopyrrolopyrrole of the formula (I) according to one of claims 1 to 4 as nonoxidative dye in colorants for keratin fibres.

## Revendications

1. Dicétopyrrolopyrrole cationique de formule générale (I) 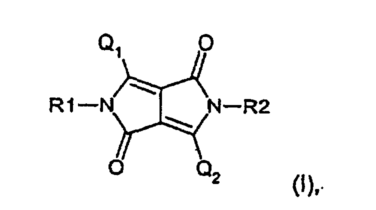 dans laquelle Q₁ et Q₂ représentent, indépendamment l'un de l'autre, un radical isocyclique ou hétérocyclique aromatique éventuellement substitué ayant de 5 à 14 atomes dans le noyau, le radical hétérocyclique renfermant au moins un atome d'oxygène, d'azote ou de soufre, et les radicaux R1 et R2 représentent, indépendamment l'un de l'autre, un hydrogène ou un groupe Y-B⁺ A⁻, à condition qu'au moins l'un des radicaux R1 et R2 ne représente pas un hydrogène ; B⁺ représente un groupe ammonium quaternaire ou un groupe phosphonium quaternaire, aromatique, aliphatique, alicyclique, hétérocyclique aromatique ou hétérocyclique non aromatique ; Y représente un groupe alkylène en C₁ à C₆ linéaire ou ramifié, éventuellement substitué et A⁻ représente un anion.

2. Dicétopyrrolopyrrole selon la revendication 1, **caractérisé en ce que** dans la formule (I), B⁺ représente (i) un composé ammonium quaternaire hétérocyclique aromatique ; (ii) un composé ammonium quaternaire hétérocyclique non aromatique ; (iii) un composé alkylammonium, un composé arylammonium ou un composé arylalkylammonium quaternaire de formule NR⁵R⁶R⁷, dans laquelle R⁵, R⁶ et R⁷ représentent, indépendamment les uns des autres, un radical benzyle, un radical phényle ou un radical alkyle en C₁ à C₆, les radicaux alkyle susmentionnés pouvant être non substitués ou substitués par un ou plusieurs groupes hydroxy ou groupes amino ; ou (iv) un composé phosphonium quaternaire.

3. Dicétopyrrolopyrrole selon la revendication 1 ou 2, **caractérisé en ce que** dans la formule (I), Y représente un groupe -CH₂, un groupe -CH₂-CH₂, un groupe -CH₂-CH₂-CH₂, un groupe -CH₂-CH₂-CH₂-CH₂ ou un radical alkylène en C₂à C₆ linéaire ou ramifié, substitué par un ou plusieurs groupes alkyle, groupes hydroxy, groupes amino, groupes acyle ou groupes ammonium quaternaires.

4. Dicétopyrrolopyrrole selon Tune quelconque des revendications 1 à 3, **caractérisé en ce que** dans la formule (I), l'anion A⁻ est choisi parmi un iodure, un chlorure, un bromure, un sulfate, un phosphate, un hydrogénophosphate, un oxalate, un carbonate, un hydrogénocarbonate, un formiate, un acétate, un citrate, un tartrate, un malonate et un pyruvate.

5. Procédé de préparation des dicétopyrrolopyrroles de formule (I) selon l'une quelconque des revendications 1 à 4, dans lequel un composé de formule (II) est mis à réagir avec le dihalogénure d'alkyle correspondant en présence d'une base dans un solvant polaire aprotique, et l'halogénure obtenu est ensuite mis à réagir avec une amine tertiaire ou une phosphine tertiaire.

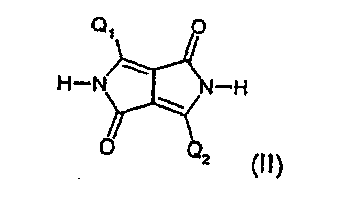

6. Procédé de préparation des dicétopyrrolopyrroles de formule (I) selon l'une quelconque des revendications 1 à 4, dans lequel un composé de formule (II) 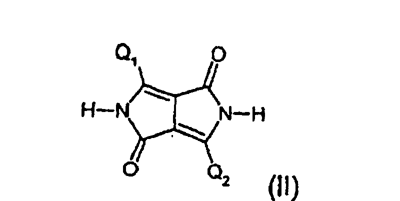 est mis à réagir avec une chaîne cationique préformée X-Y-B⁺ A⁻.

7. Composition pour la teinture de fibres kératiniques, **caractérisée en ce qu'**elle comprend au moins un dicétopyrrolopyrrole de formule (I) selon l'une quelconque des revendications 1 à 4.

8. Composition selon la revendication 7, **caractérisée en ce que** le dicétopyrrolopyrrole de formule (I) est choisi parmi le dibromure de 2,5-bis(4-triméthylammoniumbutyl)-3,6-diphénylpyrrolo[3,4-c]pyrrole-1,4-dione, le dichlorure de 2,5-bis(4-triméthylammoniumbutyl)-3,6-diphényl-pyrrolo[3,4-c]pyrrole-1,4-dione, le dibromure de 2,5-bis(4-triméthylammoniumbutyl)-3,6-bis[4-(diméthylamino)phényl]pyrrolo[3,4-c]pyrrole-1,4-dione, le dichlorure de 2,5-bis(4-triméthylammoniumbutyl)-3,6-bis[4-(diméthylamino)phényl]pyrrolo[3,4-c]pyrrole-1,4-dione, le dibromure de 2,5-bis[4-[4-(diméthylamino)pyridinium]butyl]-3,6-diphénylpyrrolo[3,4-c]pyrrole-1,4-dione ; le dichlorure de 2,5-bis[4-[4-(diméthylamino)pyridinium]butyl]-3,6-diphénylpyrrolo[3,4-c]pyrrole-1,4-dione ; le dibromure de 2,5-bis[4-[N-(méthylimidazolium)butyl]]-3,6-diphényl-pyrrolo[3,4-c]pyrrole-1,4-dione et le dichlorure de 2,5-bis[4-[N-(méthylimidazolium)butyl]]-3,6-diphénylpyrrolo[3,4-c]pyrrole-1,4-dione.

9. Composition selon la revendication 7 ou 8, **caractérisée en ce que** le dicétopyrrolopyrrole de formule (I) est présent en une quantité de 0,01 à 10 pourcent en poids.

10. Composition selon l'une quelconque des revendications 7 à 9, **caractérisée en ce qu'**elle comprend en outre également au moins un autre colorant direct parmi le groupe constitué de colorants végétaux, de colorants nitro, de colorants azoïques, de colorants anthraquinone et de colorants triphénylméthane.

11. Composition selon l'une quelconque des revendications 7 à 10, **caractérisée en ce qu'**il s'agit d'une composition de teinture capillaire.

12. Utilisation d'un dicétopyrrolopyrrole de formule (I) selon l'une quelconque des revendications 1 à 4 en tant que colorant non oxydant dans des compositions de teinture pour des fibres kératiniques.
